# EUROPEAN PATENT APPLICATION

(11) **EP 4 600 351 A1**
(43) Date of publication of application: **13.08.2025**
(21) Application number: 23875098.8
(22) Date of filing: 13.09.2023
(51) Int. Cl.: C12N 5/078

(54) **METHOD FOR PRODUCING PLATELETS**

(30) Priority: 05.10.2022 KR 20220127105; 29.03.2023 KR 20230041264
(71) Applicant: Dewcell, Inc., Seongnam-si, Gyeonggi-do 13494 (KR)
(72) Inventor: KIM, Chi Hwa, Gwangju 61991 (KR); HWANG, Chi Yeon, Anyang-si, Gyeonggi-do 14022 (KR)
(74) Representative: Klöckner, Christoph
(86) International application number: PCT/KR2023/013756
(87) International publication number: WO 2024/076030

(57) **Abstract**

The present invention relates to a method for producing platelets. The method of the present invention includes the steps of: (S1) culturing pluripotent stem cells to obtain a culture medium containing hematopoietic stem cells; (S2) obtaining a suspended cell population from the culture medium when the cells in the culture medium that do not express CD41a constitute at least 15% of the total cells; and (S3) differentiating the suspended cell population into megakaryocytes, whereby the method enables the production of high-quality platelets with a high yield.

## Description

### CROSS-REFERENCE TO RELATED APPLICATION AND CLAIM OF PRIORITY

This application claims priority to Korean Patent Application Nos. 10-2022-0127105 and 10-2023-0041264 filed on October 05, 2022, and March 29, 2023, respectively, in the Korean Intellectual Property Office (KIPO), the entire disclosure of which is incorporated by reference herein.

### TECHINICAL FIELD

The present invention relates to a method for producing platelets. This specification was prepared with the support of the following national research project.

**[Table 1]**

| Category | National Research Project 1 | National Research Project 2 |
|---|---|---|
| Project Number | 1465040518 | 1415187966 |
| Ministry | Ministry of Health and Welfare | Ministry of Trade, Industry and Energy |
| Research Management Organization | (Foundation) Korean Fund for Regenerative Medicine | Korea Institute for Advancement of Technology |
| Research Program Name | Development of translational technology in regenerative medicine | Materials and Components Technology Development project |
| Research Project Name | Clinical study approval of stem cell-derived i-aPLP^{™} for osteoarthritis therapy | Development of artificial human platelet lysate (i-ahPLys^{™}) to substitute for fetal bovine serum (FBS) |
| Sponsor | DewCell, Inc. | DewCell, Inc. |
| Research Period | 2023.04.01 - 2026.12.31 | 2023.04.01-2025.12.31 |

### BACKGROUND

Platelet preparations are administered to patients with massive bleeding during surgery or injury, or bleeding associated with thrombocytopenia after chemotherapy, for the purpose of treating and preventing these symptoms.

Currently, the production of platelet preparations relies on blood donations from healthy volunteers. Recently, the number of blood donors has been decreasing, and a future shortage of blood is anticipated. Therefore, the stable supply of platelets is an important issue in this technical field.

Conventional platelet preparations have a high risk of bacterial contamination, which can lead to serious infections after platelet transfusion. Therefore, there is a constant demand in clinical settings for safer platelet preparations.

To meet this demand, methods for producing platelets from megakaryocytes cultured *in vitro* have recently been developed. However, platelets are not yet obtained in high yields, and the process is not economically viable, requiring technical improvements.

### SUMMARY

An object of the present invention is to provide a method for producing artificial platelets in high yield.

Another object of the present invention is to provide a method for producing platelets with high process efficiency.
1. A method for producing platelets, comprising: (S1) culturing pluripotent stem cells to obtain a culture containing hematopoietic stem cells; (S2) obtaining a floating cell population from the culture when the number of CD41a⁻ cells among the cells contained in the culture is at least 15% of the total number of cells; and (S3) differentiating the floating cell population into megakaryocytes in a first medium.
2. The method for producing platelets according to above 1, further comprising (S4) culturing and maturing the megakaryocytes in a second medium containing thrombopoietin.
3. The method for producing platelets according to above 1, wherein (S1) comprises the following steps: (S1a) culturing the pluripotent stem cells in a third medium containing a GSK3 inhibitor; (S1b) culturing the cells cultured in the third medium in a fourth medium containing vascular endothelial growth factor and basic fibroblast growth factor; and (S1c) culturing the cells cultured in the fourth medium in a fifth medium containing vascular endothelial growth factor; basic fibroblast growth factor; and a transforming growth factor beta signaling inhibitor.
4. The method for producing platelets according to above 1, wherein the floating cell population has a CD45⁺ cell count of 50% or less of the total cell count.
5. The method for producing platelets according to above 1, wherein the floating cell population has a CD34⁺ cell count of 45% or more of the total cell count.
6. The method for producing platelets according to above 1, wherein the floating cell population has a CD41a⁻ cell count of 85% or less of the total cell count.
7. The method for producing platelets according to above 1, further comprising (S0) seeding the pluripotent stem cells on the bottom of a culture vessel at 2,000 to 20,000 cells/cm².
8. The method for producing platelets according to above 1, wherein the culture further comprises hematopoietic progenitor cells and megakaryocyte progenitor cells.
9. The method for producing platelets according to above 1, wherein the first medium comprises thrombopoietin, stem cell factor, interleukin-3, and interleukin-6.
10. The method for producing platelets according to above 1, wherein, in (S2), the floating cell population is obtained from the culture when the number of CD41a⁺ cells among the cells contained in the culture is 40 to 85% of the total number of cells.
11. The method for producing platelets according to above 1, wherein, in (S2), the floating cell population is obtained from the culture when the number of CD34⁺ cells among the cells contained in the culture is 45 to 80% of the total number of cells.
12. The method for producing platelets according to above 1, wherein, in (S2), the floating cell population is obtained from the culture when the number of CD45⁺ cells among the cells contained in the culture is 1 to 50% of the total number of cells.
13. The method for producing platelets according to above 1, wherein the pluripotent stem cells are human induced pluripotent stem cells.
14. The method for producing platelets according to above 4, wherein the floating cell population has a proportion of CD45⁺ cells lower than a proportion of CD41a+ cells.
15. A method for producing a blood product, comprising a step of mixing platelets produced by the method of any one of above 1 to 14 with other blood components.

The method for producing platelets of the present invention does not require the separation and purification of CD41a⁺ cells because megakaryocytes are produced from a floating cell population containing at least 15% CD41a⁻ cells.

The method for producing platelets of the present invention enables rapid and economical platelet production because the subsequent process can be performed when the floating cell population satisfies certain requirements.

The method for producing platelets of the present invention has excellent differentiation efficiency into megakaryocytes and platelet production efficiency.

The method for producing platelets of the present invention is simple, does not require complex equipment, and is advantageous in terms of time and cost.

Platelets produced according to the method of the present invention can be activated by stimulants (e.g., ADP (adenosine diphosphate), collagen, fibrinogen, etc.) to increase PAC-1 and CD62p expression. This confirms that the platelets of the present invention are platelets with normal function.

Platelets produced according to the method of the present invention can exhibit a therapeutic or preventive effect on diseases related to platelet reduction or dysfunction.

### BRIEF DESCRIPTION OF THE DRAWINGS

FIG. 1 is an embodiment of the culture process of human induced pluripotent stem cells.
FIG. 2 is a result of confirming the surface markers (CD34, CD45, CD41a) of the floating cell populations of Preparation Examples 1-1 to 1-4 and Comparative Preparation Example 1-1.
FIG. 3 is a result of confirming the surface markers (CD34, CD45, CD41a) of the floating cell populations of Preparation Examples 1-1 to 1-3 and Comparative Preparation Example 1-1.
FIG. 4 is a process for obtaining the cell cultures of Examples 1 and 2.
FIG. 5 is a photograph of the cell culture of Example 1.
FIG. 6 is a process for obtaining the cell culture of Comparative Example 1.
FIG. 7 is a photograph of the cell culture of Comparative Example 1.
FIG. 8 shows that the cells of Example 1 express megakaryocyte-specific markers.
FIG. 9 shows that the cells of Example 2 express megakaryocyte-specific markers.
FIG. 10 shows that the cells of Comparative Example 1 do not express megakaryocyte-specific markers.
FIG. 11 shows that the cells of Example 1 were activated by ADP treatment, and PAC-1 and CD62p expression was increased.
FIG. 12 shows that the cells of Example 2 were activated by ADP treatment, and PAC-1 and CD62p expression was increased.
FIG. 13 is a result of selecting CD41a⁺ cells in the floating cell population of Preparation Example 1-2.
FIG. 14 shows the ratio of CD41a⁺/CD42b⁺ in Example 1 (Preparation Example 1-2) and Comparative Example 2.
FIG. 15 shows that Example 1 exhibits about 40 times higher cell proliferation ability than Comparative Example 2.
FIG. 16 is a result of confirming the surface markers (CD34, CD45, CD41a) of the floating cell populations of Preparation Examples 3-1 to 3-6 and Comparative Preparation Examples 3-1 and 3-2.
FIG. 17 to FIG. 22 show that Examples 3 to 8 express megakaryocyte and platelet-specific markers.
FIG. 23 and 24 show that Comparative Examples 3 and 4 do not express megakaryocyte and platelet-specific markers.

### DETAILED DESCRIPTION OF THE EMBODIMENTS

The present invention provides a method for producing platelets comprising the following steps: (S1) culturing pluripotent stem cells to obtain a culture containing hematopoietic stem cells; (S2) obtaining a floating cell population from the culture when the number of cells not expressing CD41a among the cells contained in the culture is at least 15% of the total number of cells; and (S3) differentiating the floating cell population into megakaryocytes in a first medium.

The production method of the present invention may further comprise (S0) seeding the pluripotent stem cells on the bottom of a culture vessel at 2,000 to 20,000 cells/cm².

The production method of the present invention may further comprise (S4) culturing and maturing the megakaryocytes in a second medium containing thrombopoietin.

The production method of the present invention may further comprise (S5) obtaining platelets from the culture obtained in (S4).

Hereinafter, the platelet production process from (S0) to (S5) will be described.

### (S0) Step

(S0) is a step of seeding pluripotent stem cells on the bottom of a culture vessel at 2,000 to 20,000 cells/cm².

Pluripotent stem cells include induced pluripotent stem cells (iPSC) or embryonic stem cells (ESC).

Induced pluripotent stem cells (iPSC) refer to cells that did not have pluripotency but have acquired pluripotency through an artificial reprogramming process.

Induced pluripotent stem cells (iPSC) may be derived from individuals selected from the group consisting of humans, non-human primates, rodents (mice, rats), ungulates (cattle, sheep, etc.), canines (domestic and wild dogs), felines (domestic and wild cats such as lions, tigers, cheetahs), rabbits, hamsters, goats, elephants, pandas (including giant pandas), pigs, raccoons, horses, zebras, and marine mammals (dolphins, whales, etc.).

Induced pluripotent stem cells may be human induced pluripotent stem cells (hiPSC).

Induced pluripotent stem cells may be generated using mouse and/or human cells. For example, induced pluripotent stem cells may be generated using embryonic, fetal, neonatal, and adult tissues.

Induced pluripotent stem cells may be derived from virtually any somatic cell at any developmental stage as a starting point. Somatic cells may be, but are not limited to, derived from embryonic, fetal, neonatal, juvenile, or adult donors. Somatic cells may be, but are not limited to, fibroblasts, such as skin fibroblasts obtained by skin sample or biopsy, synoviocytes from synovial tissue, buccal cells, or lung fibroblasts.

Pluripotent stem cells are attached and cultured after being seeded on the bottom of the culture vessel.

The culture vessel can be of any type without limitation as long as it is used for cell culture in the art. For example, it may be a large, medium, or small culture vessel. It may also be a cell culture flask such as T25, T75, T175, or T225.

Seeding pluripotent stem cells at 2,000 to 20,000 cells/cm² is preferable in terms of cell confluency. When seeded in this amount, cell confluency may be 70% to 95%, 75% to 95%, 80% to 95%, or 85% to 95%, and the pluripotent stem cells are properly cultured and differentiated, so that a culture containing a sufficient amount of hematopoietic stem cells, hematopoietic progenitor cells, megakaryocyte progenitor cells, and the like can be obtained in the (S1) step described later.

If pluripotent stem cells are seeded at less than 2,000 cells/cm² on the bottom of the culture vessel, cell confluency may be 70% or less, 65% or less, 60% or less, 55% or less, 50% or less, 45% or less, 40% or less, 35% or less, 30% or less, etc. at the time of obtaining the floating cell population of (S2). In addition, if pluripotent stem cells are seeded at more than 20,000 cells/cm² on the bottom of the culture vessel, cell confluency may exceed 100% at a time when the pluripotent stem cells have not been sufficiently cultured and differentiated, and undifferentiated cells may detach from the culture dish and become floating and die, or the amount of factors added to the culture may be insufficient, resulting in poor signaling between the cells and poor differentiation.

### (S1) Step

(S1) is a step of culturing pluripotent stem cells to obtain a culture containing hematopoietic stem cells. This step is a step of culturing the pluripotent stem cells seeded in the (S0) step to obtain a floating cell population that can be differentiated into megakaryocytes.

The (S1) step may be configured to include the following sub-steps:
(S1a) culturing the pluripotent stem cells in a third medium containing a GSK3 (Glycogen Synthase Kinase 3) inhibitor;
(S1b) culturing the cells cultured in the third medium in a fourth medium containing vascular endothelial growth factor (VEGF) and basic fibroblast growth factor (bFGF); and
(S1c) culturing the cells cultured in the fourth medium in a fifth medium containing vascular endothelial growth factor; basic fibroblast growth factor; and a transforming growth factor beta signaling inhibitor.

The third medium, fourth medium, and fifth medium used in this step are media having different compositions and purposes.

The third medium at least contains a GSK3 inhibitor. The GSK3 inhibitor may be, but is not limited to, CHIR99021, BIO (6-bromoindirubin-30-oxime), SB216763, CHIR-98014, CT98014, CT98023, CT99021, TWS119, SB41528, AR-A014418, AZD-1080, Alsterpaullone, Cazpaullone, or Kenpaullone. CHIR99021 is a GSK3 inhibitor, a Wnt signaling enhancer, and may be represented as an aminopyrimidine.

The GSK3 inhibitor (e.g., CHIR99021) is not limited to a specific concentration as long as it is in an amount capable of culturing pluripotent stem cells. The GSK3 inhibitor may be included in the third medium at a concentration of, for example, 2 to 10 µM, 2.5 to 9.5 µM, 3 to 9 µM, 3.5 to 8.5 µM, 4 to 8 µM, 4.5 to 7.5 µM, 5 to 7 µM, 5.5 to 6.5 µM, or 6 µM.

The third medium is a basal medium. The third medium may comprise an RPMI1640 medium or another type of basal medium. The third medium may further comprise an antioxidant and/or B-27 in addition to the basal medium.

The antioxidant may be selected from the group consisting of 6-hydroxymelatonin, acetyl-L-carnitine (ALCAR), alpha-lipoic acid (ALA), ascorbic acid (e.g., AA2P (L-Ascorbic acid 2-phosphate), AA2G (L-Ascorbic acid 2-glucoside)), carotenoids (vitamin A), curcumin, edaravone, polyphenols, glutathione, hydroxytyrosol, L-camitine, ladostigil, melatonin, mofegiline, N-acetylcysteine (NAC), N-acetylserotonin (NAS), oleocanthal, oleuropein, rasagiline, resveratrol, selegiline, selenium, tocopherols (vitamin E), tocotrienols, tyrosol, ubiquinone (coenzyme Q), and uric acid.

The fourth medium is a growth medium. The fourth medium at least contains growth factors such as vascular endothelial growth factor (VEGF) and basic fibroblast growth factor (bFGF). The fourth medium may further contain other growth factors to culture pluripotent stem cells.

Vascular endothelial growth factor (VEGF) is a member of the epidermal growth factor receptor (EGFR/ErbB). Vascular endothelial growth factor plays an essential role in regulating cell proliferation and differentiation, and leads to the activation of various forms of signaling pathways to induce apoptosis, survival, or cell proliferation. Vascular endothelial growth factor includes, for example, human and non-human animal (e.g., mouse) vascular endothelial growth factor.

Vascular endothelial growth factor is included at a concentration capable of appropriately culturing pluripotent stem cells. Vascular endothelial growth factor may be included in the fourth medium at a concentration of, for example, 5 to 95 ng/ml, 10 to 90 ng/ml, 15 to 85 ng/ml, 20 to 80 ng/ml, 25 to 75 ng/ml, 30 to 70 ng/ml, 35 to 65 ng/ml, 40 to 60 ng/ml, 45 to 55 ng/ml, or 50 ng/ml.

Basic fibroblast growth factor (bFGF) is a protein belonging to the FGF family that functions as a mitogenic factor, angiogenesis factor, bone formation factor, and nerve growth factor in cell proliferation, cell differentiation, and more. Basic fibroblast growth factor, also called FGF2, activates receptor proteins including FGFR1b, FGFR1c, FGFR2c, FGFR3c, FGFR4c and the like, and in particular strongly activates FGFR1c and FGFR3c.

Basic fibroblast growth factor is included at a concentration capable of appropriately culturing pluripotent stem cells together with other components. Basic fibroblast growth factor may be included in the fourth medium at a concentration of, for example, 1 to 40 ng/ml, 5 to 35 ng/ml, 10 to 30 ng/ml, 15 to 25 ng/ml, or 20 ng/ml.

The fourth medium may not contain a transforming growth factor beta (TGFβ) signaling inhibitor. If the fourth medium contains a TGFβ signaling inhibitor, differentiation may occur without sufficient cell proliferation.

The fifth medium is a growth medium. Like the fourth medium, it at least contains growth factors such as vascular endothelial growth factor (VEGF) and basic fibroblast growth factor (bFGF). The fifth medium may further contain other growth factors to culture pluripotent stem cells.

The matters relating to vascular endothelial growth factor (VEGF) and basic fibroblast growth factor (bFGF) in the fifth medium are the same as those for vascular endothelial growth factor (VEGF) and basic fibroblast growth factor (bFGF) in the fourth medium.

The fifth medium contains a transforming growth factor beta (TGFβ) signaling inhibitor.

Transforming growth factor beta (TGFβ) signaling inhibitor refers to a substance that inhibits TGFβ signaling. TGFβ is a substance that regulates various physiological processes in vivo, such as cell proliferation, differentiation, apoptosis, migration, production of extracellular matrix (ECM), angiogenesis, and development.

The TGFβ signaling inhibitor can be of any type without limitation as long as it is a substance capable of inhibiting TGFβ signaling, and may be, for example, an activin receptor-like kinase (ALK) receptor inhibitor.

The activin receptor-like kinase receptor inhibitor may be, but is not limited to, an ALK5, ALK4, and ALK7 receptor inhibitor. For example, the ALK receptor inhibitor may be SB431542. SB431542 may be expressed by the following compound name: 4-[4-(2H-1,3-benzodioxol-5-yl)-5-(pyridin-2-yl)-1H-imidazol-2-yl] benzamide.

The transforming growth factor beta (TGFβ) signaling inhibitor is included at a concentration capable of culturing pluripotent stem cells together with other components. The transforming growth factor beta (TGFβ) signaling inhibitor may be included at a concentration of, for example, 1 to 20 µM, 5 to 15 µM, or 10 µM.

The culture obtained through the culture of the (S1) step contains hematopoietic stem cells (HSC). The culture may further contain hematopoietic progenitor cells (HPC) and megakaryocyte progenitor cells (MK-P).

### (S2) Step

(S2) is a step of obtaining a floating cell population from the culture of (S1) when the number of cells not expressing CD41a (CD41a⁻ cells) among the cells contained in the culture is at least 15% of the total number of cells.

The floating cell population refers to cells and/or cell populations floating in the culture. The floating cell population contains a large number of cells that can be differentiated into megakaryocytes, such as hematopoietic stem cells, hematopoietic progenitor cells, and megakaryocyte progenitor cells, and may contain cells that are not directly differentiated into megakaryocytes, but help hematopoietic stem cells, hematopoietic progenitor cells, and megakaryocyte progenitor cells to differentiate better than when they are alone.

This step determines the optimal time to obtain the floating cell population from the culture of (S1). Through this step, by obtaining the optimal floating cell population for differentiation into megakaryocytes, the differentiation efficiency into megakaryocytes and/or matured megakaryocytes and the platelet production efficiency can be increased.

The floating cell population is obtained when the number of CD41a⁻ cells is at least 15% of the total number of cells. That is, the number of cells expressing CD41a (CD41a⁺ cells) is less than 85%. If the number of CD41a⁺ cells is less than 85%, the differentiation efficiency into megakaryocytes and platelet production efficiency are excellent, and if the number of CD41a⁺ cells is 85% or more, the platelet production efficiency is rather lowered.

The number of CD41a⁻ cells is, for example, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84% of the total number of cells.

The number of CD41a⁻ cells is for example, 85% or less, 84% or less, 83% or less, 82% or less, 81% or less, 80% or less, 79% or less, 78% or less, 77% or less, 76% or less, 75% or less, 74% or less, 73% or less, 72% or less, 71% or less, 70% or less, 69% or less, 68% or less, 67% or less, 66% or less, 65% or less, 64% or less, 63% or less, 62% or less, 61% or less, 60% or less, 59% or less, 58% or less, 57% or less, 56% or less, 55% or less, 54% or less, 53% or less, 52% or less, 51% or less, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31% or less, 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, or 15% or less of the total number of cells.

The percentage of CD41a⁺ cells can be obtained by subtracting the percentage of CD41a⁻ cells from 100%.

In (S2), it is preferable that the number of CD41a⁺ cells among the cells contained in the culture is 15% or more and less than 85%, 20% or more and less than 85%, 30% or more and less than 85%, 40% or more and less than 85%, 45% or more and less than 85%, or 50% or more and less than 85% of the total number of cells.

Along with whether the cells in the culture express CD41a, the time to obtain the floating cell population can be determined in consideration of the number of cells expressing CD45. The floating cell population can be obtained from the culture when the number of CD45⁺ cells is, for example, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, or 35% or less of the total number of cells.

In addition, the floating cell population can be obtained from the culture when the number of CD45⁺ cells is, for example, 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, or 20% or more.

In one embodiment, the number of CD45+ cells is 1 to 50%, 5 to 50%, 10 to 50%, or 15 to 50%.

Along with whether the cells of the culture express CD41a, the time to obtain the floating cell population can be determined in consideration of the number of cells expressing CD34. The floating cell population can be obtained from the culture when the number of CD34⁺ cells is, for example, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, or 75% or more.

In addition, the floating cell population can be obtained from the culture when the number of CD34⁺ cells is, for example, 90% or less, 89% or less, 88% or less, 87% or less, 86% or less, 85% or less, 84% or less, 83% or less, 82% or less, 81% or less, 80% or less, 79% or less, 78% or less, 77% or less, 76% or less, 75% or less, 74% or less, 73% or less, 72% or less, 71% or less, 70% or less, 69% or less, 68% or less, 67% or less, 66% or less, 65% or less, 64% or less, 63% or less, 62% or less, 61% or less, 60% or less, 59% or less, 58% or less, 57% or less, 56% or less, 55% or less, 54% or less, 53% or less, 52% or less, 51% or less, or 50% or less.

Along with whether the cells of the culture medium express CD41a, the floating cell population can be obtained when the percentage of CD41a⁺ cells is higher than the percentage of CD45⁺ cells.

Even if the proportion of CD41a⁺ cells in the total cell population is as low as less than 50%, if the proportion of CD45⁺ cells is lower than that, differentiation into megakaryocytes and platelets can occur well.

By determining whether the cells of the culture express CD41a, as well as CD45 and CD34, it is not necessary to separately isolate or purify only cells capable of differentiating into megakaryocytes from the total cell population.

The method of counting the number of cells can use any conventional techniques in the art without limitation. For example, an antibody-based selection method or a sorter machine may be used. The antibody-based selection method may be, for example, a method using microbeads.

### (S3) Step

(S3) is a step of differentiating the floating cell population obtained in the (S2) step into megakaryocytes in a first medium.

The first medium serves to differentiate the floating cell population into megakaryocytes. The first medium may include cytokines. The first medium may include at least one of thrombopoietin (TPO), stem cell factor (SCF), interleukin-3 (IL-3), and interleukin-6 (IL-6).

Thrombopoietin is a major growth factor that regulates megakaryocyte and platelet hematopoiesis and is mainly synthesized and secreted by hepatocytes.

Thrombopoietin may be included in the first medium at a concentration capable of culturing the floating cell population together with other components, and is not required to be included at a specific concentration. Thrombopoietin may be included at a concentration of, for example, 1 to 50 ng/ml, 5 to 45 ng/ml, 10 to 40 ng/ml, 15 to 35 ng/ml, 20 to 30 ng/ml, or 25 ng/ml.

Stem cell factor is a stromal cell-derived cytokine synthesized by fibroblasts and other cell types. Stem cell factor may be included in the first medium at a concentration capable of culturing the floating cell population together with other components, and is not required to be included at a specific concentration. Thrombopoietin may be included at a concentration of, for example, 1 to 50 ng/ml, 5 to 45 ng/ml, 10 to 40 ng/ml, 15 to 35 ng/ml, 20 to 30 ng/ml, or 25 ng/ml.

Interleukin-3 may be included in the first medium at a concentration capable of culturing the floating cell population together with other components, and is not required to be included at a specific concentration. Interleukin-3 may be included at a concentration of, for example, 1 to 20 ng/ml, 5 to 15 ng/ml, or 10 ng/ml.

Interleukin-6 may be included in the first medium at a concentration capable of culturing the floating cell population together with other components, and is not required to be included at a specific concentration. Interleukin-6 may be included at a concentration of, for example, 1 to 20 ng/ml, 5 to 15 ng/ml, or 10 ng/ml.

The first medium is not limited to a specific medium. The first medium may include IMDM (Iscove's Modified Dulbecco's Medium) as a basal medium. The IMDM may further include an antioxidant (e.g., AA2P, etc.) and/or B-27.

### (S4) Step

(S4) is a step of culturing and maturing the megakaryocytes of (S3) in a second medium containing thrombopoietin.

Thrombopoietin may be included in the second medium at a concentration of, for example, 50 to 150 ng/ml, 60 to 140 ng/ml, 70 to 130 ng/ml, 80 to 120 ng/ml, 90 to 110 ng/ml, or 100 ng/ml, but is not limited thereto.

The second medium may further include factors necessary for maturing megakaryocytes in addition to thrombopoietin.

The second medium is not limited to a specific medium. The second medium may include IMDM (Iscove's Modified Dulbecco's Medium) as a basal medium. The IMDM may further include an antioxidant (e.g., Ascorbic Acid 2-Phosphate, AA2P, etc.) and/or B-27.

What is obtained in this step may be matured megakaryocytes, or a culture containing megakaryocytes and/or matured megakaryocytes.

### (S5) Step

(S5) is a step of obtaining platelets from the culture obtained in (S4).

This step may be separating and/or purifying platelets from the culture after further differentiating the culture of (S4), or separating and/or purifying platelets from the culture of (S4) without further differentiating it.

Separation and/or purification of platelets can be performed by known separation and/or purification methods in the art. For example, it can be performed by centrifuging the culture or passing the culture through a column.

When the culture is centrifuged, megakaryocytes can be separated as a precipitate and platelets as a supernatant.

Hereinafter, the present invention will be described in more detail with reference to Examples.

### Examples

### 1. Example 1, Example 2, and Comparative Example 1

### 1-1. Production of Human Induced Pluripotent Stem Cells (hiPSC)

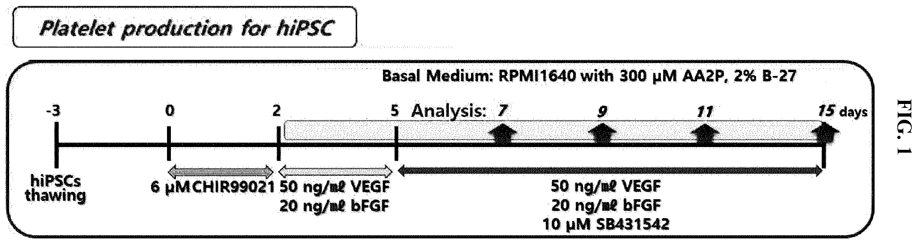

In order to obtain a cell population capable of differentiating into megakaryocytes, human induced pluripotent stem cells were cultured as follows (FIG. 1).

First, human induced pluripotent stem cells were placed in a T75 flask at a density of 4000 cells/cm² in mTeSR plus culture medium with 10 µg/ml ROCK inhibitor (Y-27632) and cultured at 37°C and 5% CO₂. 24 hours after culturing, the cells were washed once with PBS to remove Y-27632, and the mTeSR plus culture medium was replaced with fresh medium. While culture medium was replaced once a day, the cells were cultured for 3 days to obtain human induced pluripotent stem cells.

### 1-2. Production of Culture Medium Containing Human Hematopoietic Stem Cells (hHSC)

The obtained human induced pluripotent stem cells were cultured in RPMI1640 basal medium containing 300 µM AA2P and 2% B-27 with 6 µM CHIR99021, a GSK3 inhibitor, at 37°C and 5% CO₂ for 2 days (medium was replaced once a day).

Then, the medium composition was changed to RPMI1640 basal medium containing 300 µM AA2P and 2% B-27 with 50 ng/ml VEGF and 20 ng/ml bFGF, and the cells were cultured in a 37°C, 5% CO₂ incubator for 3 days (medium was replaced once a day). The culture was continued until the cell confluency was about 70% or more. Then, 10 µM SB431542 was further added to RPMI1640 basal medium containing 300 µM AA2P and 2% B-27 with VEGF and bFGF, and the cells were further cultured for 4 days.

### (1) Preparation Example 1-1

On the 2nd day of additional culture, the ratio of the number of cells expressing specific surface markers (CD34, CD41a, CD45) to the total number of floating cells was determined by flow cytometry (FACS). Lyric equipment from BD was used for flow cytometry, and anti-CD34-FITC, anti-CD45-FITC, and anti-CD41a-APC antibodies from BD were used. Through this, the ratios of single or double positive cells of CD34 and CD41a, and CD45 and CD41a were analyzed, respectively.

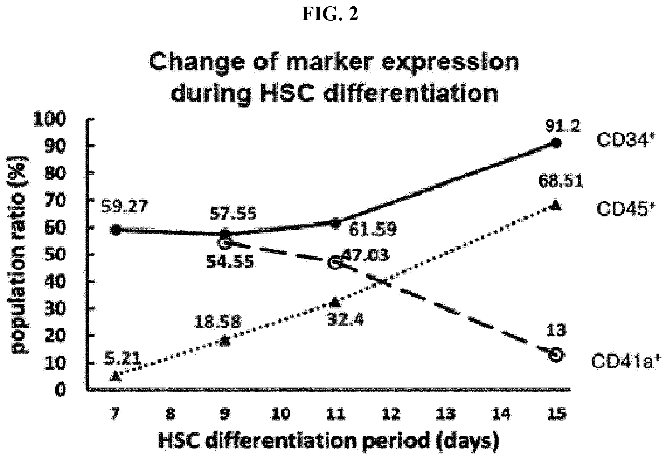

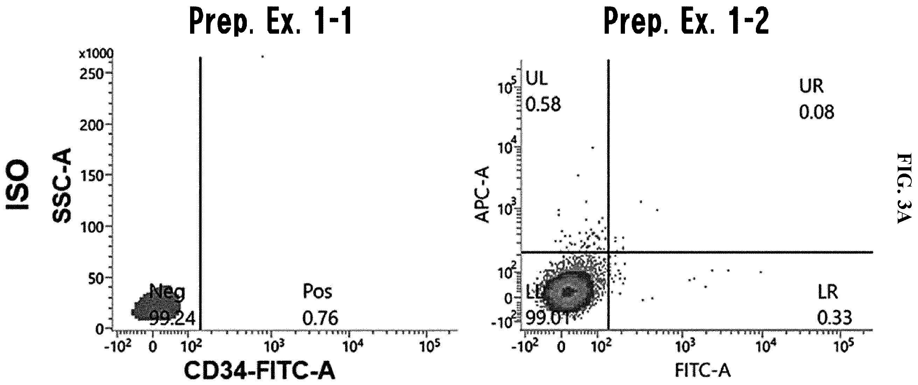

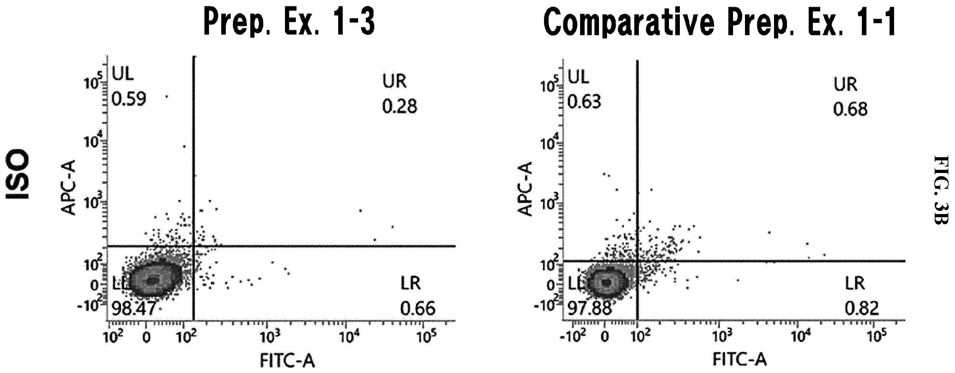

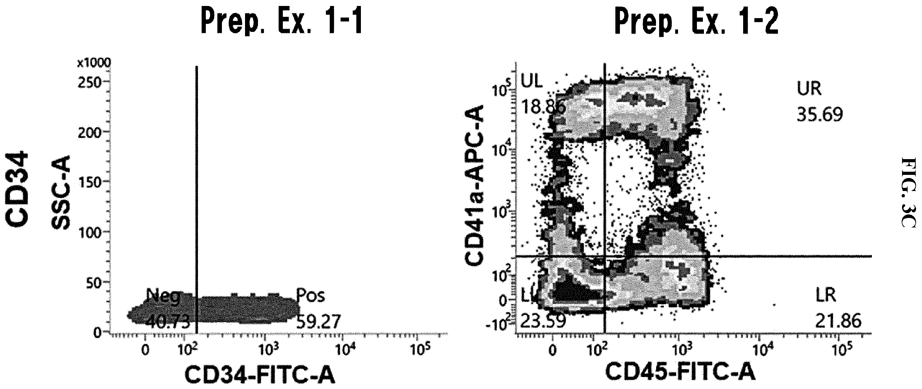

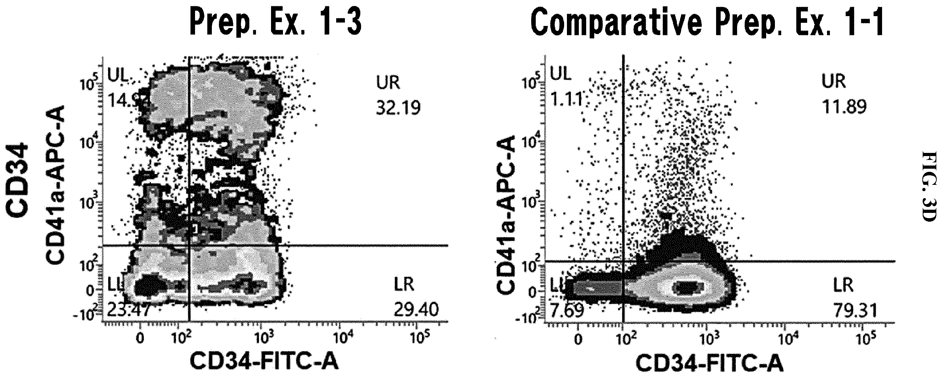

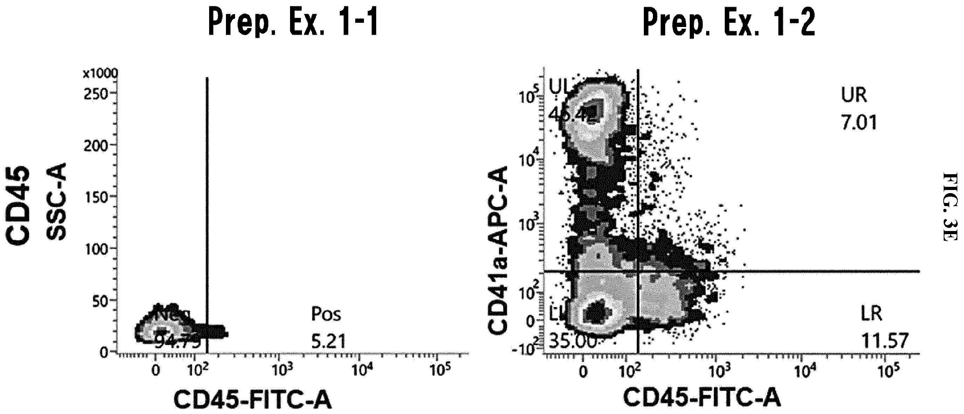

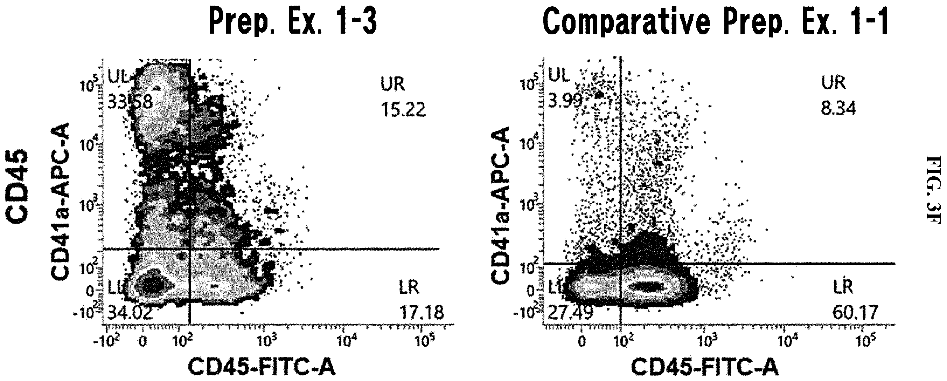

As a result, it was identified that the ratio of CD34⁺ cells to total cells was 59.27%, and the ratio of CD45⁺ cells to total cells was 5.21%, and at this point, a floating cell population was obtained from the culture (day 7 in FIG. 2 and FIG. 3).

### (2) Preparation Example 1-2

On the 4th day of additional culture, the ratio of the number of cells expressing specific surface markers (CD34, CD41a, CD45) to the total number of floating cells was determined in the same manner as in Preparation Example 1-1. As a result, it was identified that the ratio of CD34⁺ cells to total cells was 57.55%, the ratio of CD41a⁺ cells to total cells was 54.55% and the ratio of CD45⁺ cells to total cells was 18.58%, and at this point, a floating cell population was obtained from the culture (day 9 in FIG. 2 and FIG. 3).

### (3) Preparation Example 1-3

On the 6th day of additional culture, the ratio of the number of cells expressing specific surface markers (CD34, CD41a, CD45) to the total number of floating cells was determined in the same manner as in Preparation Example 1-1. As a result, it was identified that the ratio of CD34⁺ cells to total cells was 61.59%, the ratio of CD41a⁺ cells to total cells was 47.03% and the ratio of CD45⁺ cells to total cells was 32.4%, and at this point, a floating cell population was obtained from the culture (day 11 in FIG. 2 and FIG. 3).

### (4) Preparation Example 1-4

On the 8th day of additional culture, the ratio of the number of cells expressing specific surface markers (CD34, CD41a, CD45) to the total number of floating cells was determined in the same manner as in Preparation Example 1-1. As a result, it was identified that the ratio of CD34⁺ cells to total cells was 52.05%, the ratio of CD41a⁺ cells to total cells was 18.88% and the ratio of CD45⁺ cells to total cells was 45.56%, and at this time, a floating cell population was obtained from the culture (Day 13 of FIG. 2).

### (5) Comparative Preparation Example 1-1

On the 10th day of additional culture, the ratio of the number of cells expressing specific surface markers (CD34, CD41a, CD45) to the total number of floating cells was determined by flow cytometry. As a result, it was identified that the ratio of CD34⁺ cells to total cells was 91.2%, the ratio of CD41a⁺ cells to total cells was 13% and the ratio of CD45⁺ cells to total cells was 68.51%, and at this time, a floating cell population was obtained from the culture (Day 15 of FIG. 2 and FIG. 3).

### 1-3. Induction of Differentiation into Megakaryocytes by Culturing Floating Cell Population

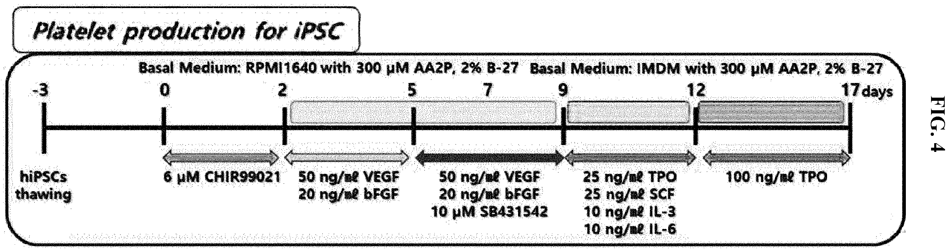

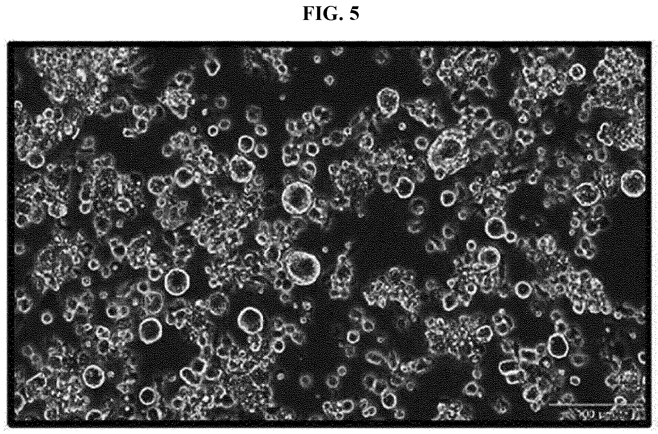

The floating cell populations from Preparation Examples 1-2 and 1-4 were cultured and introduced into IMDM basal medium containing 300 uM AA2P and 2% B-27 at a density of 1 x 10⁵ cells/ml. Then, 25 ng/ml TPO, 25 ng/ml SCF, 10 ng/ml IL-3 and 10 ng/ml IL-6 were added to the medium and the culture was incubated at 37°C and 5% CO₂ for 3 days. The medium composition was then changed to IMDM basal medium containing 300 uM AA2P and 2% B-27 with 100 ng/ml TPO, and the cultured was further incubated for 5 days to mature the megakaryocyte and obtain cell culture of Examples 1 and 2 (FIG. 4). A photograph of the cell culture of Example 1 is shown in FIG. 5.

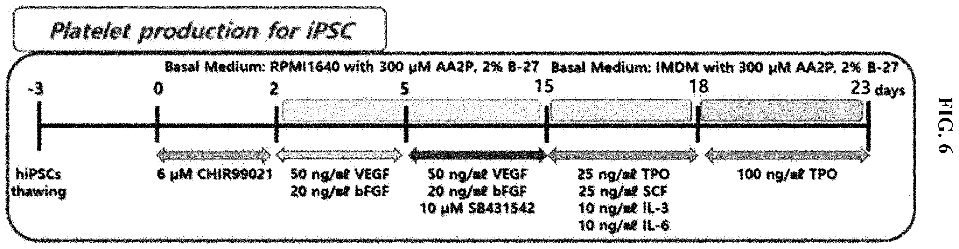

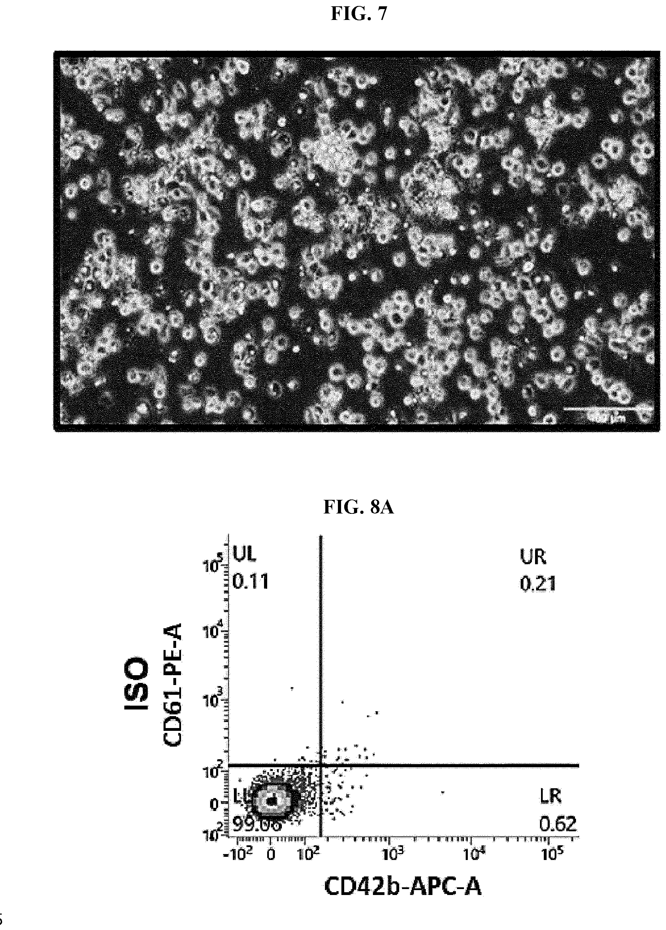

The floating cell population from Comparative Preparation Example 1-1 was cultured and introduced into IMDM basal medium containing 300 uM AA2P and 2% B-27 at a density of 1 x 10⁵ cells/ml. Then, 25 ng/ml TPO, 25 ng/ml SCF, 10 ng/ml IL-3 and 10 ng/ml IL-6 were added to the medium and the culture was incubated at 37°C and 5% CO₂ for 3 days. The medium composition was then changed to IMDM basal medium containing 300 uM AA2P and 2% B-27 with 100 ng/ml TPO, and the culture was further incubated for 5 days to mature the megakaryocyte and obtain cell culture (Comparative Example 1) (FIG. 6 and FIG. 7).

### 1-4. Confirmation of Platelet Production Ability of Megakaryocytes

The cell culture from Example 1, Example 2, and Comparative Example 1 were collected and centrifuged at 300 xg for 3 minutes. The precipitate and supernatant were separated as megakaryocytes and platelets, respectively. The platelets were then analyzed, and characterized through the following experiments.

### (1-4-1) Identify Megakaryocyte-Specific Markers Using FACS Analysis

The cell culture from Example 1, Example 2, and Comparative Example 1 were centrifuged to obtain precipitated cells (cells presumed to be megakaryocytes).

To confirm whether the precipitated cells were megakaryocytes, FACS analysis was performed to determine the surface marker expression of the precipitated cells. FACS analysis was performed using Lyric equipment from BD, and anti-CD41a-FITC, anti-CD61-PE and anti-CD42b-APC antibodies from BD were used. The antibodies were diluted 1:20 in PBS containing 1% BSA (FACS buffer), and used for a 30-minute reaction to confirm surface marker expression.

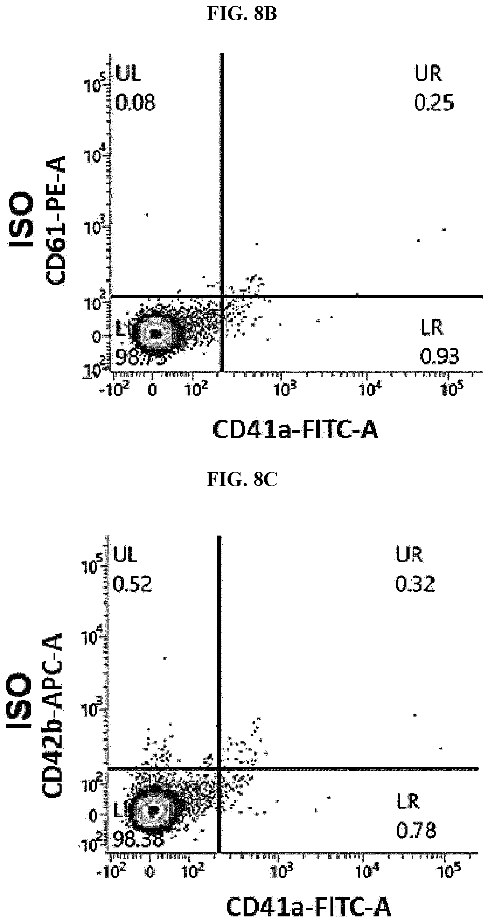

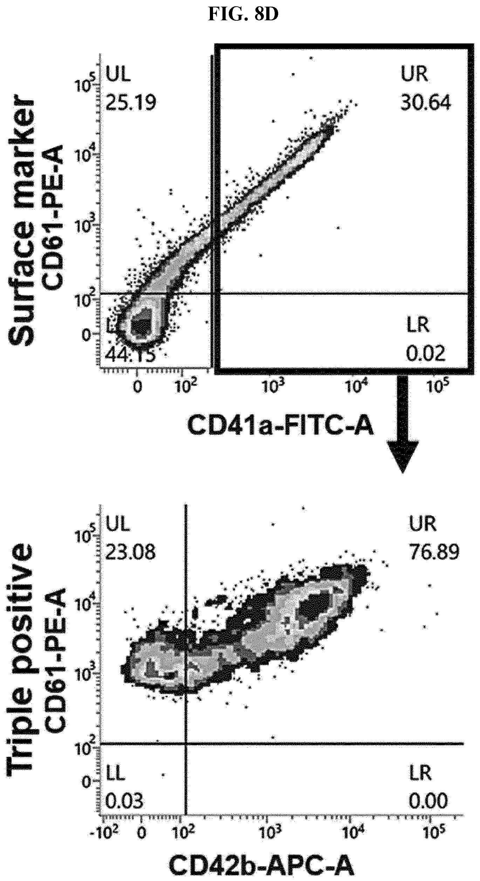

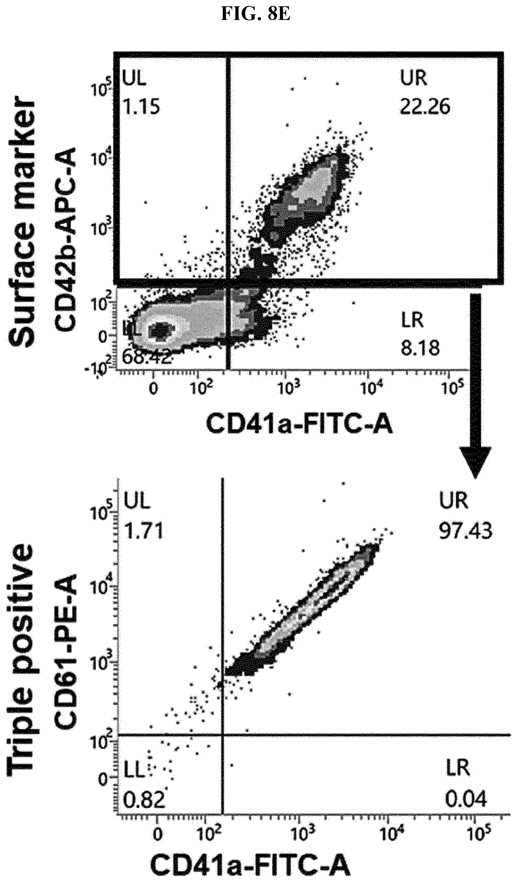

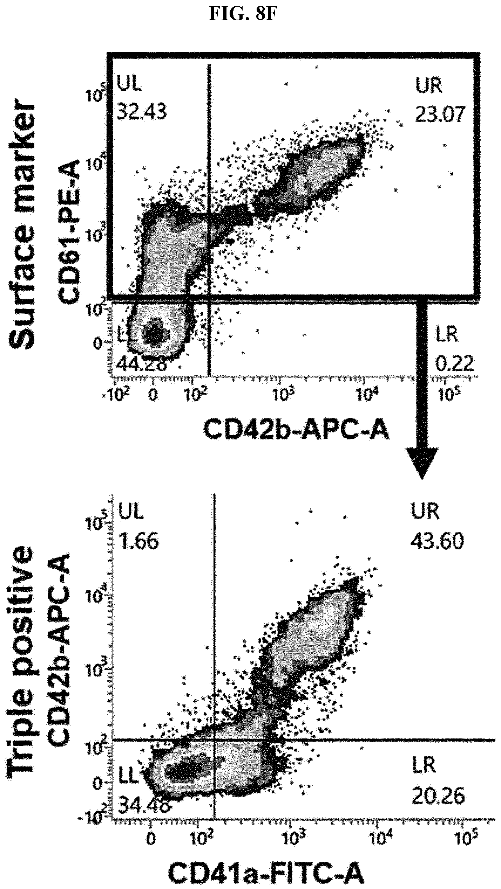

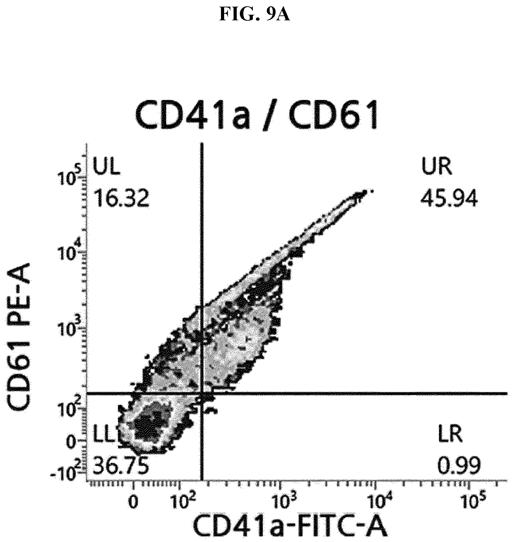

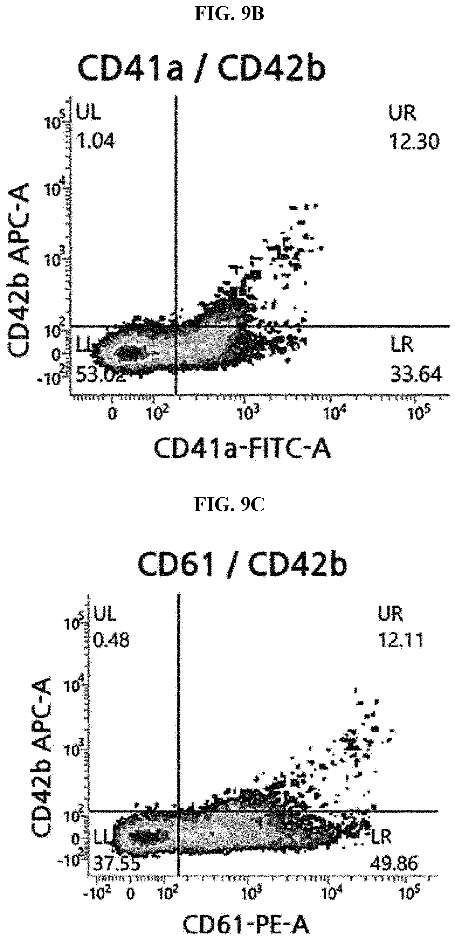

As a result, the precipitated cells from Example 1 were positive for the expression of megakaryocyte-specific markers (CD41a, CD42b, and CD61) (FIG. 8). Further, the precipitated cells from Example 2 were also positive for the expression of megakaryocyte-specific markers (CD41a, CD42b, and CD61) (FIG. 9). Therefore, it was confirmed that the floating cell populations from Examples 1 and 2 were differentiated into megakaryocytes, and accordingly, platelets were also produced.

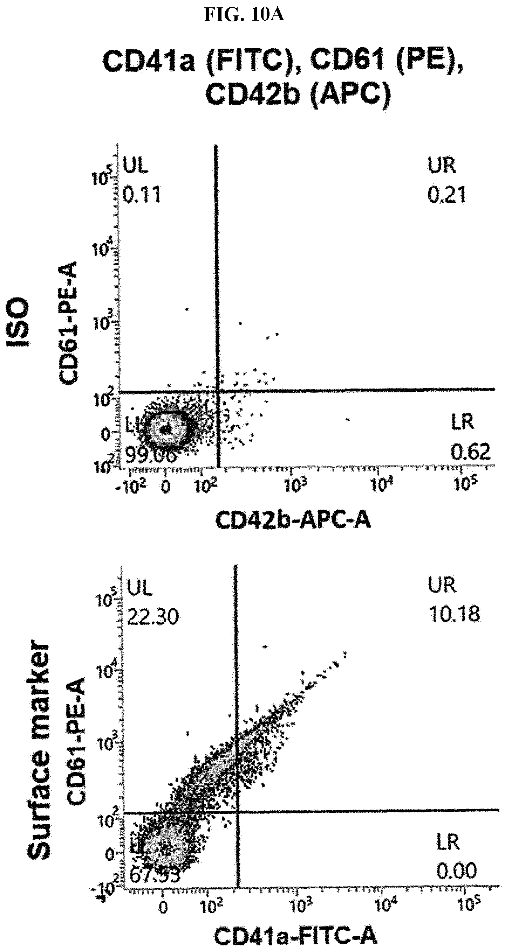

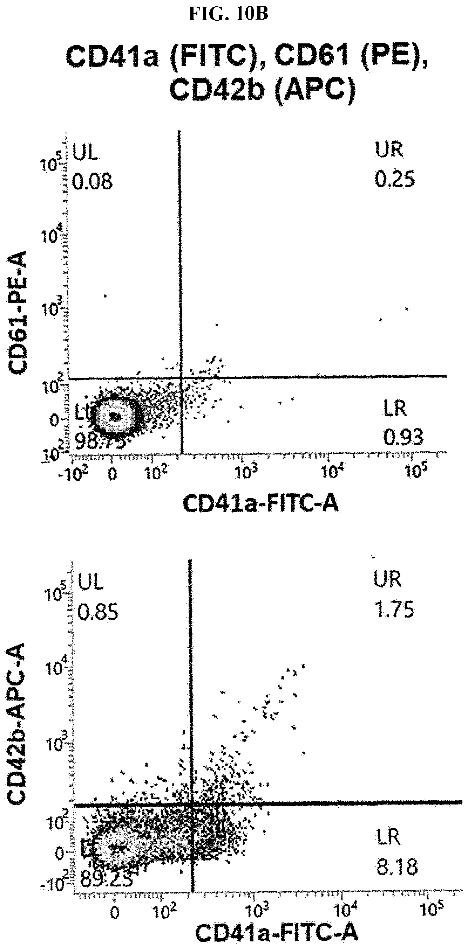

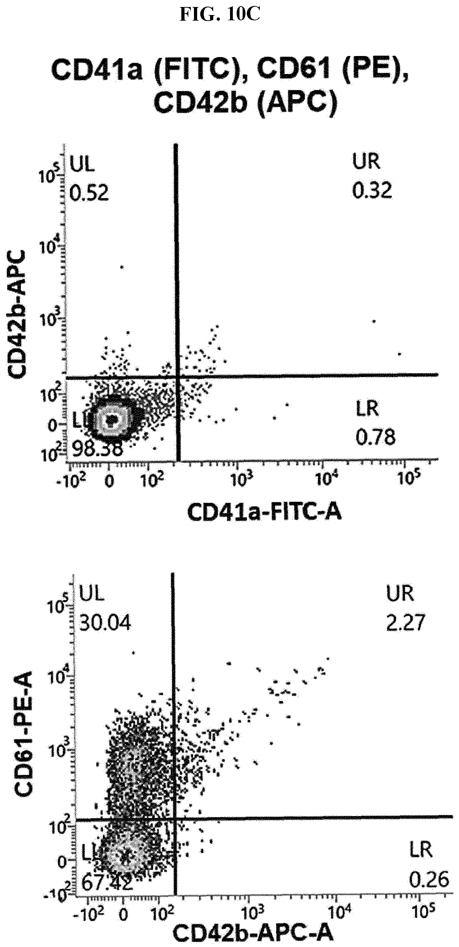

In contrast, the precipitated cells from Comparative Example 1 showed very low expression of CD41a and CD61, and especially near negative expression of CD42b (FIG. 10). This suggests that the floating cell population from Comparative Example 1 hardly differentiated into megakaryocytes, and almost no platelets were produced.

### (1-4-2) Confirmation of Platelet Function Through ADP Treatment

Example 1, Example 2, and Comparative Example 1 were centrifuged to remove precipitated cells (megakaryocytes), and the suspended cells were centrifuged again at 2000 xg for 10 minutes. The precipitated cells (cells presumed to be platelets) were then collected by removing all the supernatant, and resuspended with a small amount of PBS. The following experiment was performed to confirm whether the cells obtained in this way were platelets with normal activity.

For PAC-1 analysis, 100 µM ADP was treated to the cells and reacted at room temperature for 15 minutes, and then anti-PAC-1-FITC and anti-CD62p-PE antibodies from BD were reacted for 30 minutes. Then, FACS analysis was performed on the reacted cells.

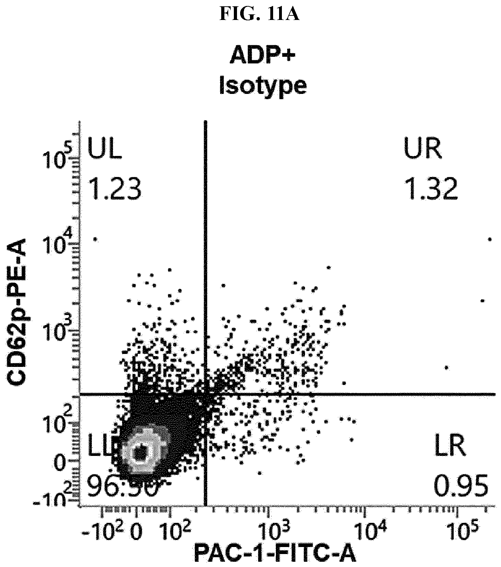

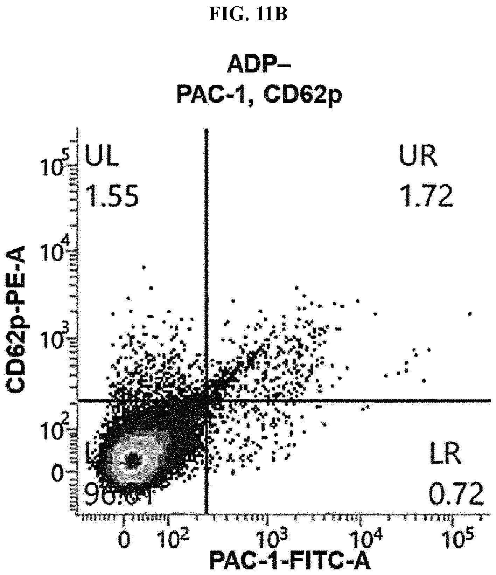

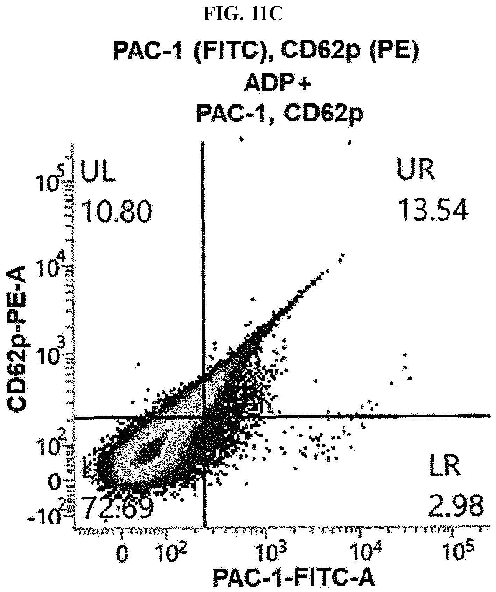

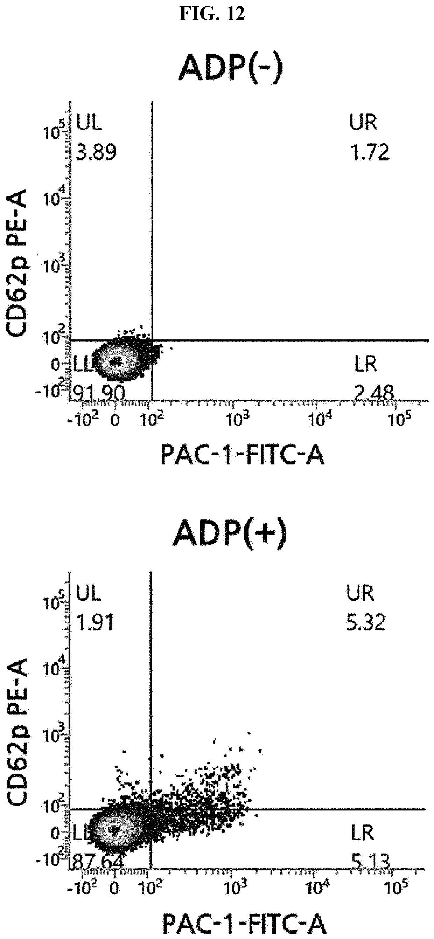

The cells from Examples 1 and 2 were activated by ADP treatment, and PAC-1 and CD62p expression was increased (FIG. 11 and FIG. 12), confirming that the cells from Examples 1 and 2 were platelets with normal activity. PAC-1 is a complex formed by CD41a and CD61 when platelets are activated. CD62p (p-selectin) is a surface molecule whose expression increases when platelets are activated and serves as a site where leukocytes can bind to platelets.

Through the above experimental results, it was confirmed that when hiPSC are cultured and the cell population satisfies certain conditions (the ratio of the number of cells expressing specific surface markers (e.g., CD41a, CD34, CD45) to the total number of cells is above or below a certain value), the differentiation efficiency into megakaryocytes and platelet production efficiency can be increased when the subsequent culture step is performed.

### 2. Comparative Example 2: Comparison of Platelet Production Efficiency When Only CD41a+ Cells Were Selected from the Floating Cell Population and Subsequently Cultured

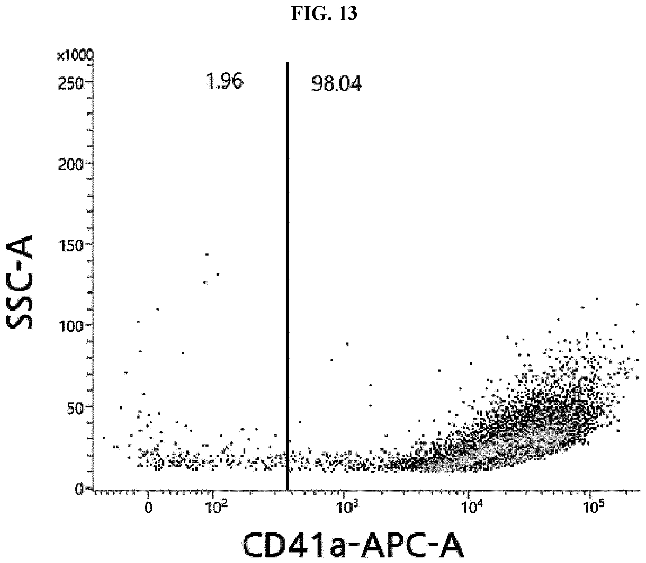

Only CD41a⁺ cells were selected from the floating cell population obtained in Preparation Example 1-2 (Comparative Example 2) and subsequently cultured, and the platelet production efficiency was compared with that of Example 1 (FIG. 13).

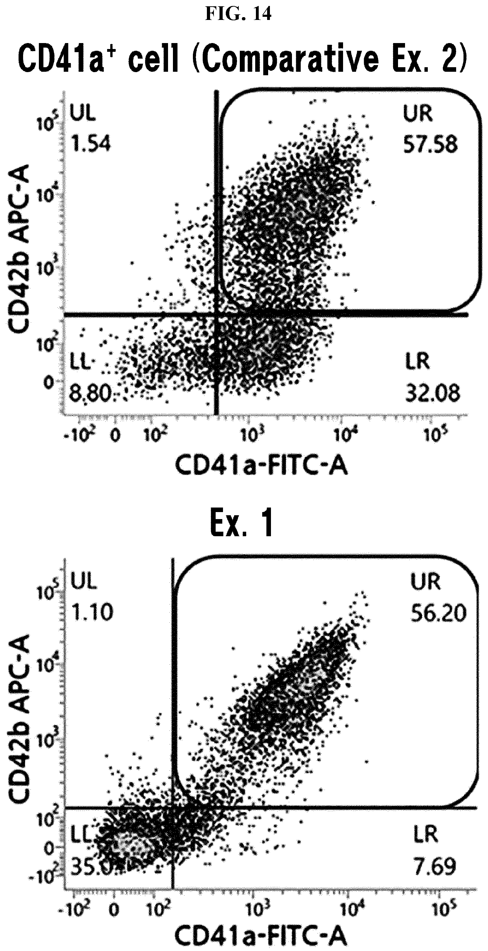

Using the same method as the above FACS analysis, the expression of platelet-specific markers in both cases was confirmed, and as a result, the ratio of CD41a⁺/CD42b⁺ was similar at 57.58 and 56.20, confirming that there was no significant difference in platelet differentiation rate (FIG. 14).

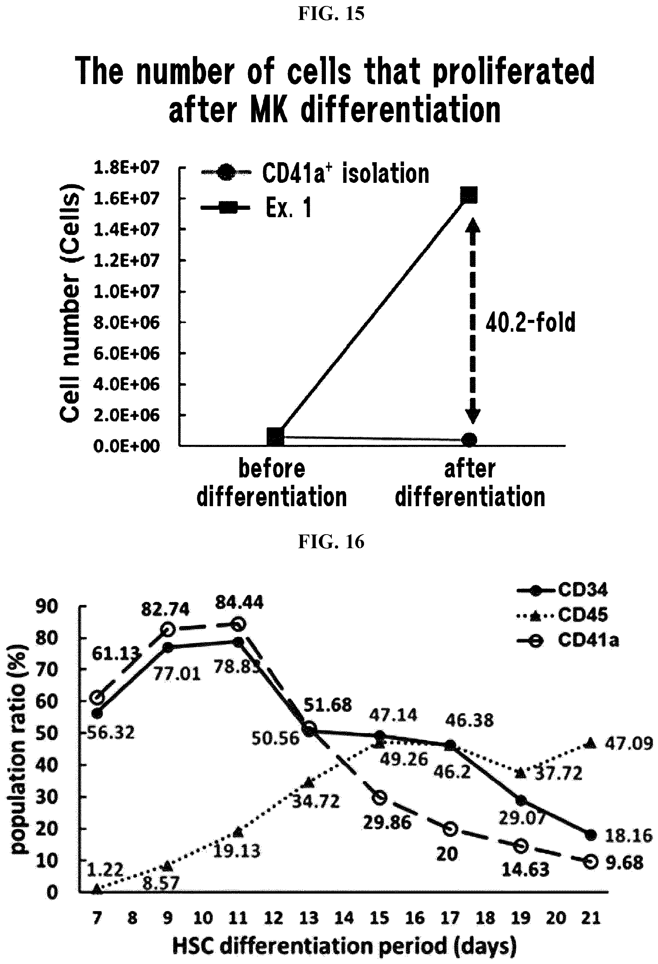

However, when the cell proliferation ability was tested, it was found that differentiation of a cell population including cells that are CD41a⁺ as well as cells that are not (e.g., CD41a⁻) into platelets, as in Example 1, showed about 40-fold higher cell proliferation ability compared to differentiation of a cell population including only CD41a⁺ cells into platelets (FIG. 15).

As a result of the above, it was confirmed that platelets can be efficiently produced quantitatively and qualitatively when a cell population including CD41a⁻ cells, as in Preparation Example 1-2, is used for platelet production, rather than selecting only CD41a⁺ cells for platelet production.

### 3. Examples 3 to 8 and Comparative Examples 3 and 4

### 3-1. Production of Human Induced Pluripotent Stem Cells (hiPSC)

In order to obtain a cell population capable of differentiating into megakaryocytes, human induced pluripotent stem cells were cultured as follows.

First, human induced pluripotent stem cells were placed in a T75 flask at a density of 4500 cells/cm² in mTeSR plus culture medium with 10 µg/ml ROCK inhibitor (Y-27632) and cultured at 37°C and 5% CO₂. 24 hours after culturing, the cells were washed once with PBS to remove Y-27632, and the mTeSR plus culture medium was replaced with fresh medium. While the culture medium was replaced once a day, the cells were cultured for 3 days to obtain human induced pluripotent stem cells.

### 3-2. Production of Culture Containing Human Hematopoietic Stem Cells (hHSC)

The obtained human induced pluripotent stem cells were cultured in RPMI1640 basal medium containing 300 µM AA2P and 2% B-27 with 6 µM CHIR99021, a GSK3 inhibitor, at 37°C and 5% CO₂ for 2 days.

Then, the medium composition was changed to RPMI1640 basal medium containing 300 µM AA2P and 2% B-27 with 50 ng/ml VEGF and 20 ng/ml bFGF, and the cells were cultured in a 37°C, 5% CO₂ incubator for 3 days. The culture was continued until the cell confluency was about 90% or more. Then, 10 µM SB431542 was further added to RPMI1640 basal medium containing 300 µM AA2P and 2% B-27 with VEGF and bFGF, and the cells were further cultured for 4 days.

The cultures were obtained on days 7, 9, 11, 13, 15, 17, 19, and 21 of culture, and the number of cells expressing specific surface markers (CD34, CD41a, and CD45) to the total number of floating cells was determined by flow cytometry (FACS). Lyric equipment from BD, and anti-CD34-FITC, anti-CD45-FITC and anti-CD41a-APC antibodies from BD were used for flow cytometry (FIG. 16).

### 3-3. Percentage of CD41a⁺, CD45⁺, and CD34⁺ Cells in the Culture

The percentages of CD41a⁺, CD45⁺, and CD34⁺ cells in the culture on days 7, 9, 11, 13, 15, 17, 19, and 21 of culture are shown in Table 2 below.

**[Table 2]**

| Category (%) | Culture Day | CD41a⁺ Cell Percentage (%) | CD45+ Cell Percentage (%) | CD34+ Cell Percentage (%) |
|---|---|---|---|---|
| Preparation Example 3-1 | 7 | 61.13 | 1.22 | 56.32 |
| Preparation Example 3-2 | 9 | 82.74 | 8.57 | 77.01 |
| Preparation Example 3-3 | 11 | 84.44 | 19.13 | 78.83 |
| Preparation Example 3-4 | 13 | 51.68 | 34.72 | 50.56 |
| Preparation Example 3-5 | 15 | 29.86 | 49.26 | 47.14 |
| Preparation Example 3-6 | 17 | 20.00 | 46.20 | 46.38 |
| Comparative Prep. Ex. 3-1 | 19 | 14.63 | 37.72 | 29.07 |
| Comparative Prep. Ex. 3-2 | 21 | 9.68 | 47.09 | 18.16 |

### 3-4. Differentiation of Floating Cell Populations of Preparation Examples 3-1 to 3-6 and Comparative Preparation Examples 3-1 and 3-2 into Megakaryocytes

The floating cell populations of Preparation Examples 3-1 to 3-6 and Comparative Preparation Examples 3-1 and 3-2 were introduced into IMDM basal medium containing 300 µM AA2P and 2% B-27 at a density of 0.5 x 10⁵ cells/ml, respectively, and cultured at 37°C and 5% CO₂ for 3 days after adding 25 ng/ml SCF, 10 ng/ml IL-3, and 10 ng/ml IL-6 to the medium.

Then, the medium composition was changed to IMDM basal medium containing 300 µM AA2P and 2% B-27 with 100 ng/ml TPO, and the cells were further cultured for 5 days to mature the megakaryocyte and obtain cell culture.

### 3-5. Confirmation of Platelet Production Ability of Examples 3 to 8 and Comparative Examples 3 and 4

The cell culture of Preparation Examples 3-1 to 3-6 and Comparative Preparation Examples 3-1 and 3-2 that had undergone the megakaryocyte maturation step were collected and centrifuged at 300 xg for 3 minutes. The precipitate, which was considered to be megakaryocytes, was removed, and the supernatant containing platelets was subjected to the following experiment to confirm whether the final product was platelets with normal activity. The substances obtained from the cell culture of Preparation Examples 3-1 to 3-6 and Comparative Preparation Examples 3-1 and 3-2 were named Examples 3 to 8 and Comparative Examples 3 and 4, respectively, in the order described.

### (1) FACS Analysis to Identify Platelet-Specific Markers

The cell culture from Preparation Examples 3-1 to 3-6 and Comparative Preparation Examples 3-1 and 3-2, which had undergone the megakaryocyte maturation step, were centrifuged. The precipitated megakaryocytes were removed. The suspended platelets were centrifuged again at 2000 xg for 15 minutes.

FACS analysis was performed on the cells, presumed to be megakaryocytes and platelets, to confirm the expression of surface markers. Lyric equipment from BD was used, and anti-CD41a-FITC, anti-CD61-PE and anti-CD42b-APC antibodies from BD were used. The antibodies were diluted 1:20 in PBS containing 1% BSA (FACS buffer), and used for a 30-minute reaction to confirm surface marker expression.

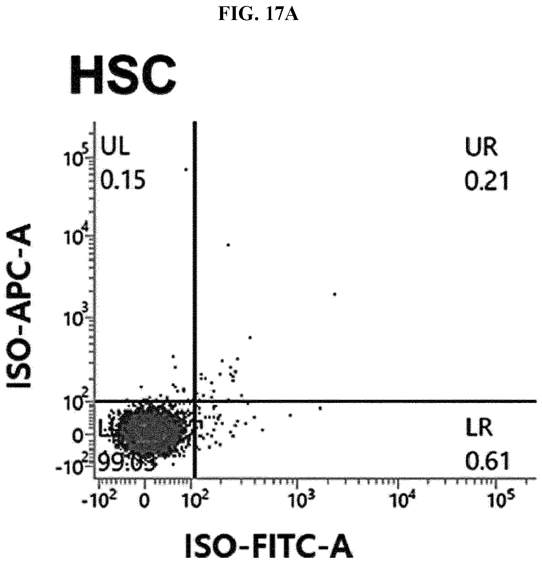

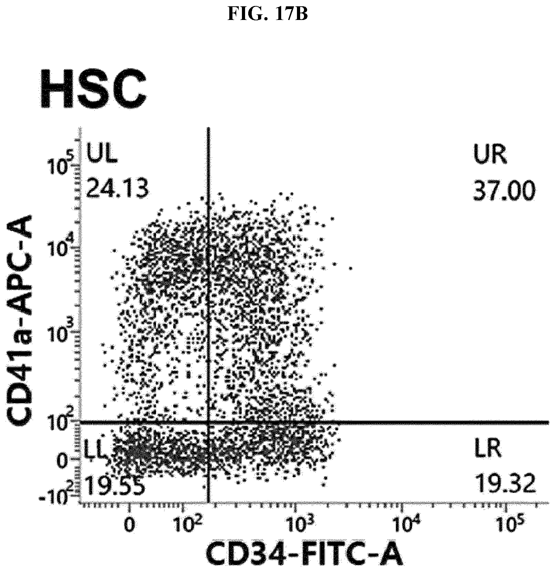

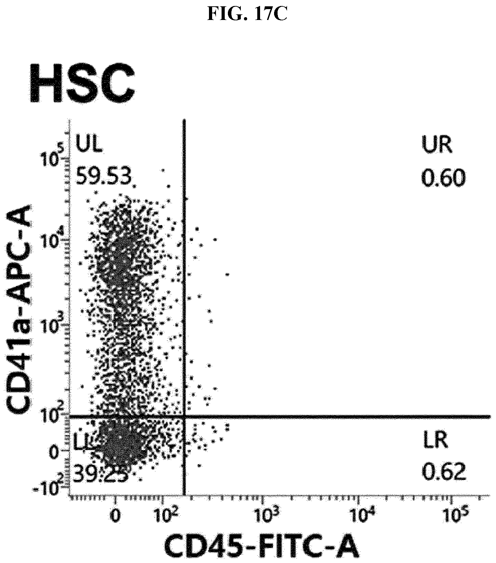

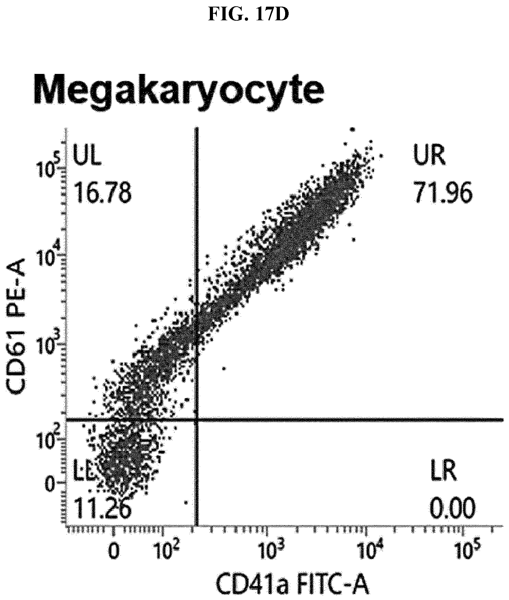

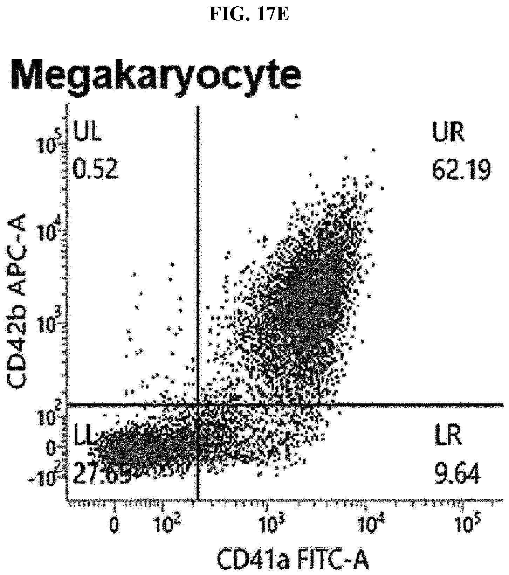

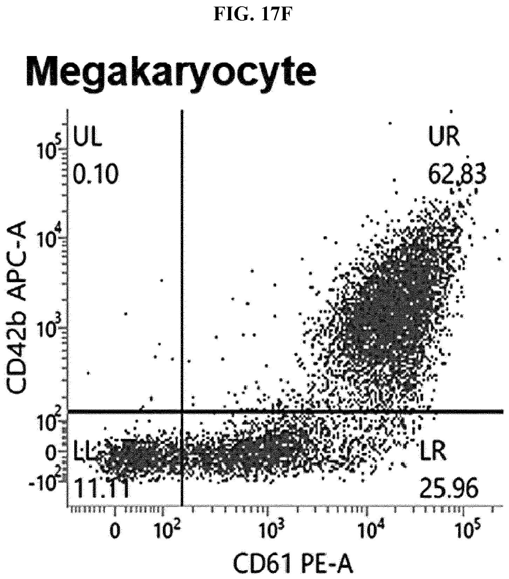

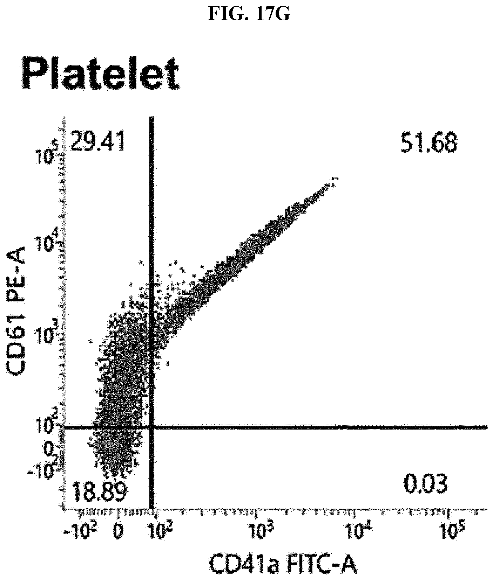

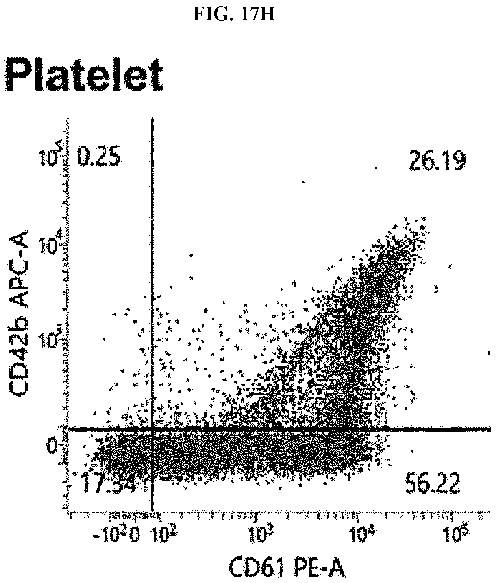

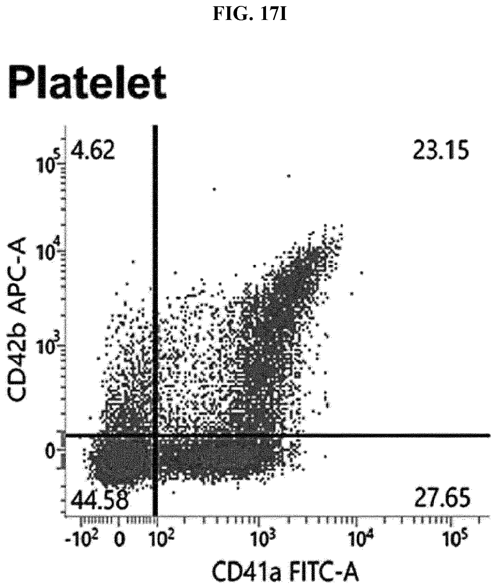

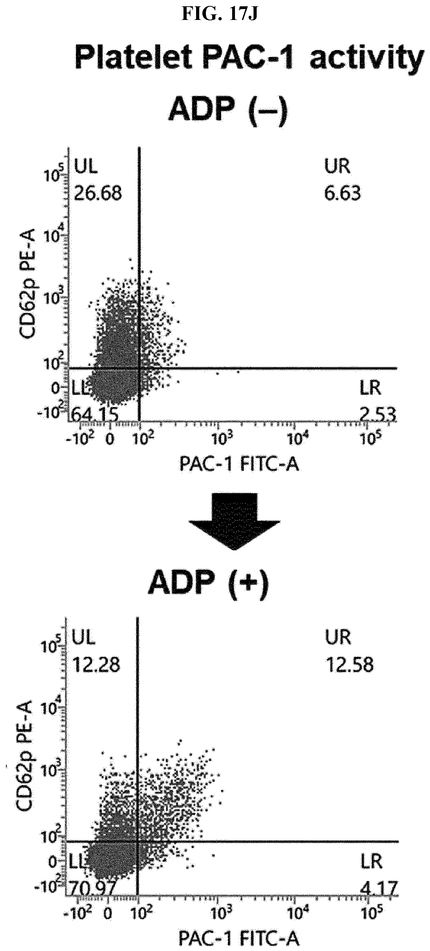

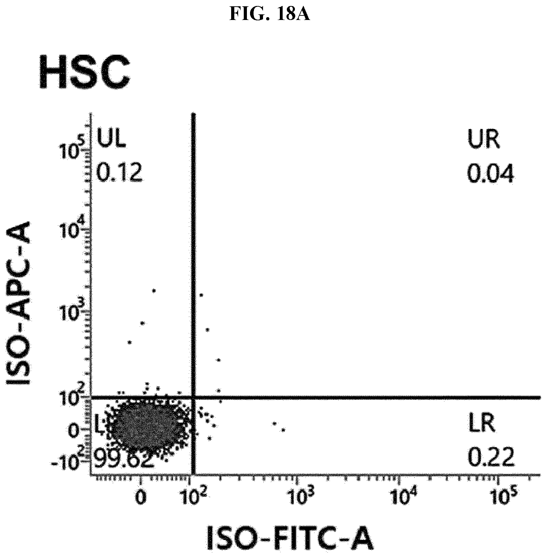

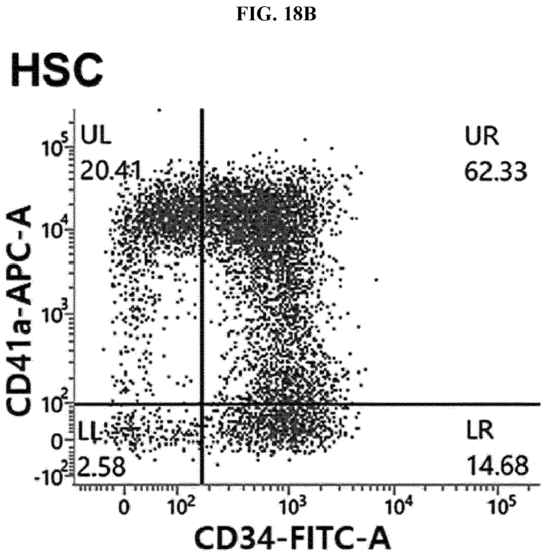

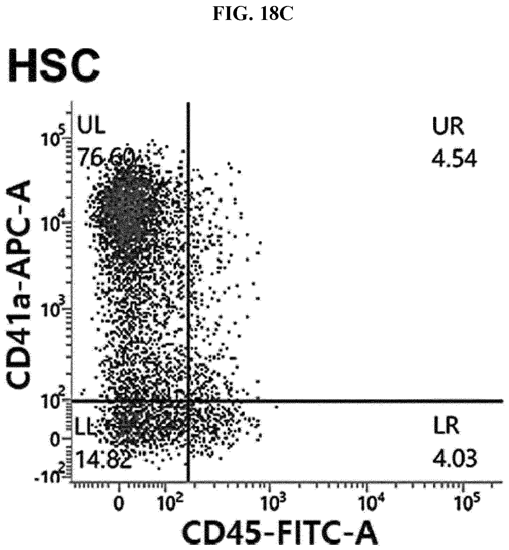

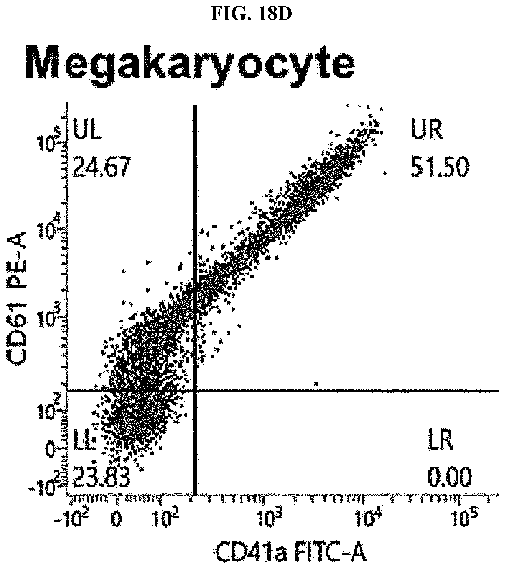

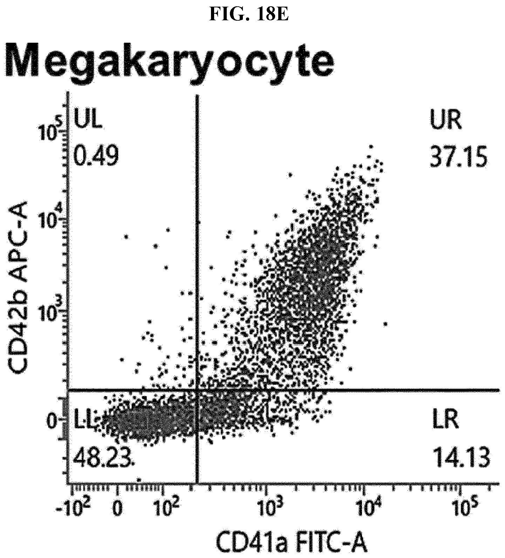

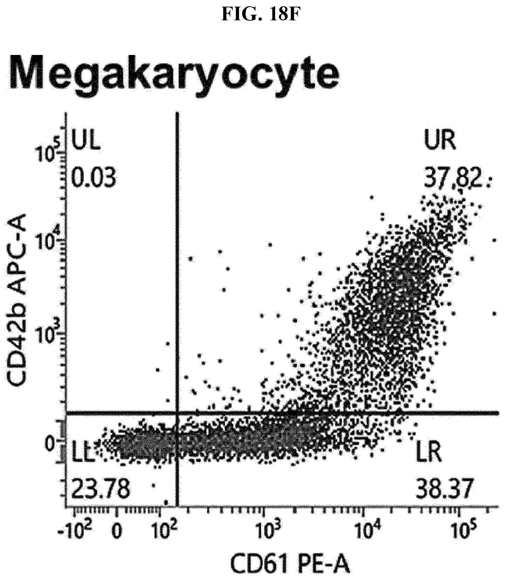

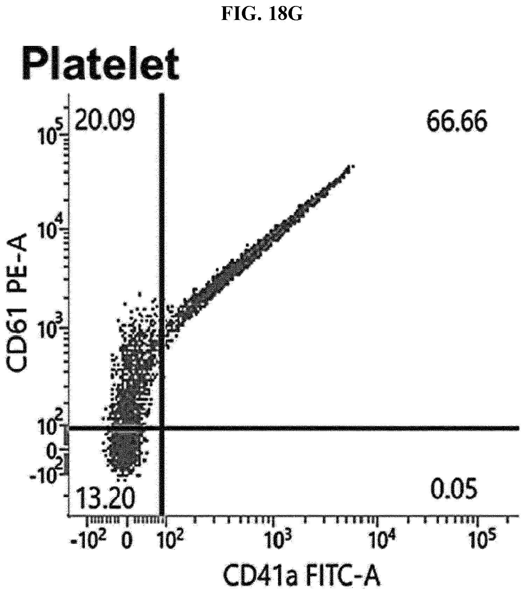

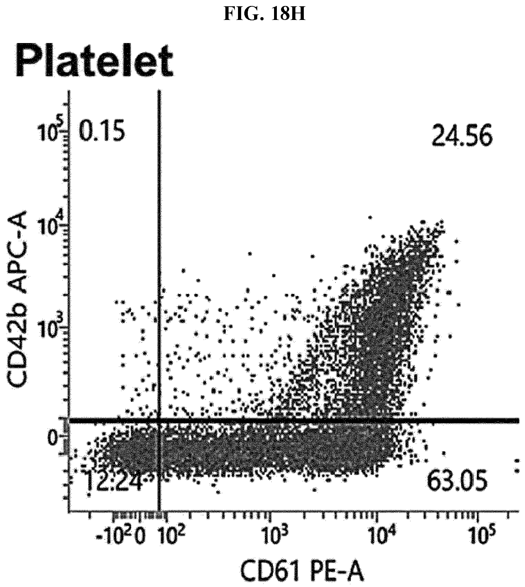

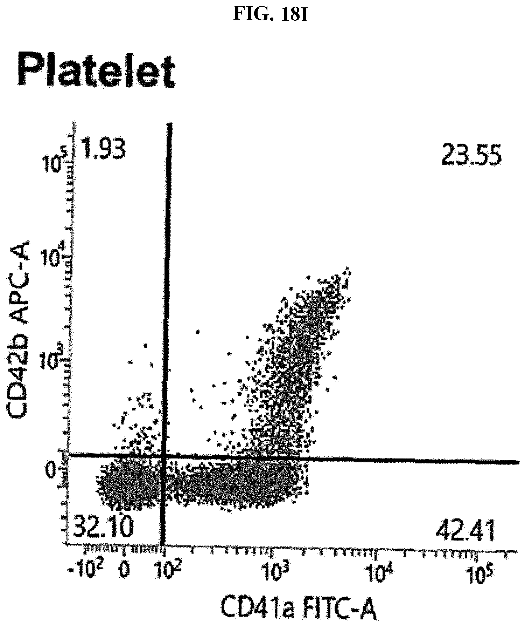

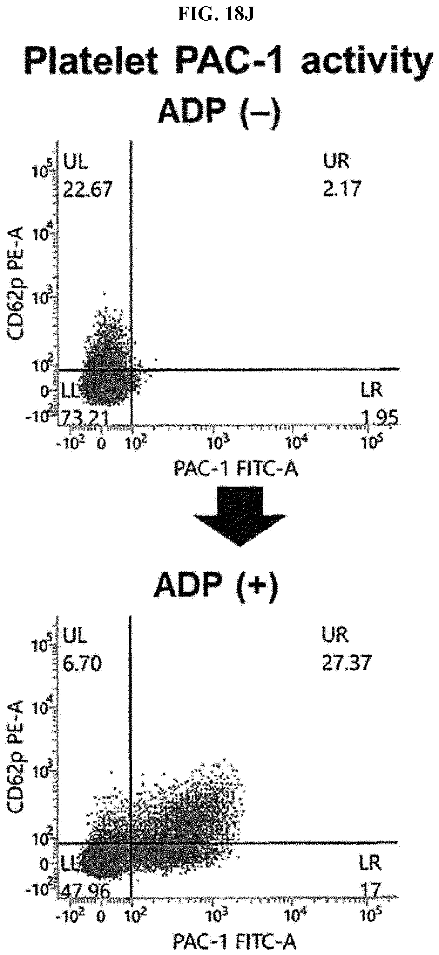

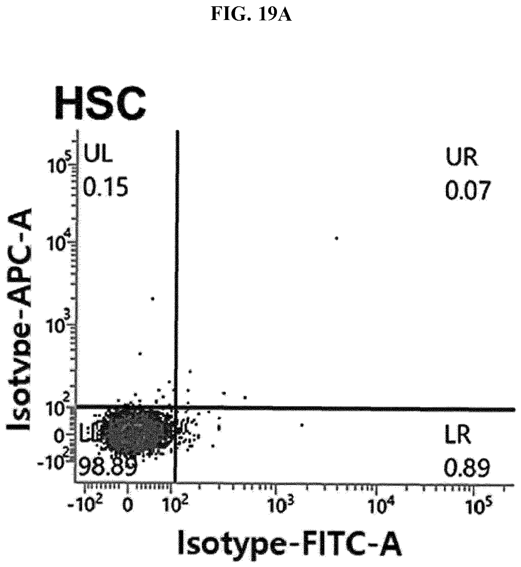

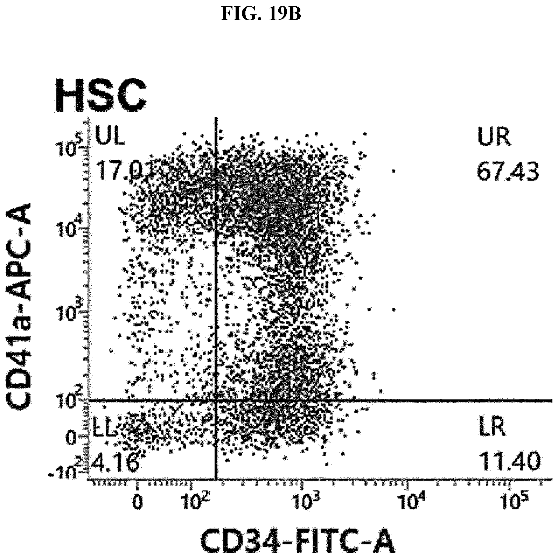

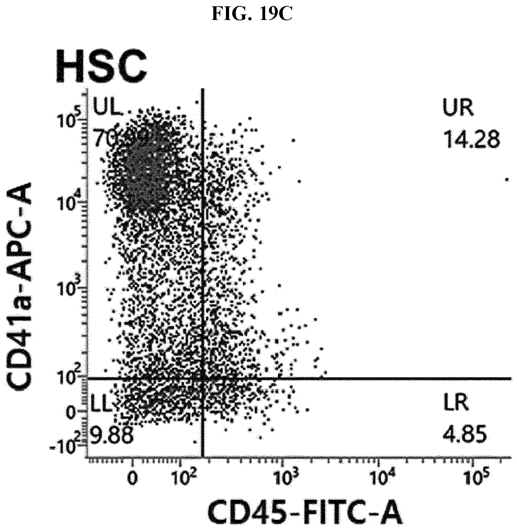

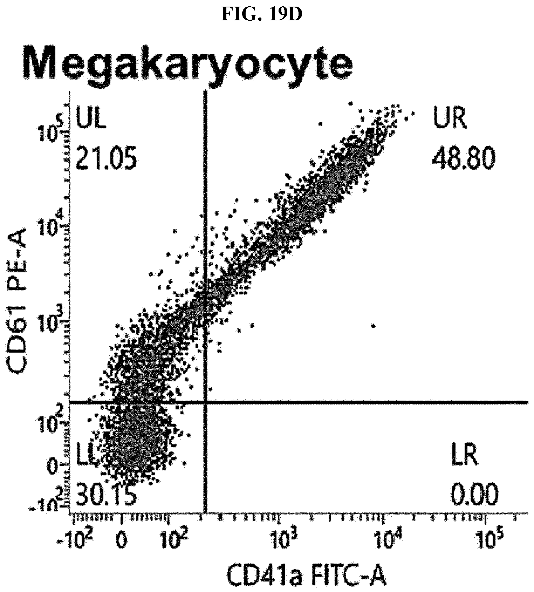

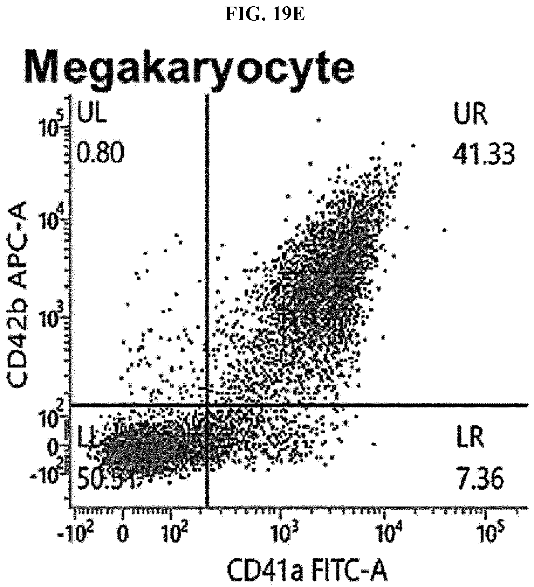

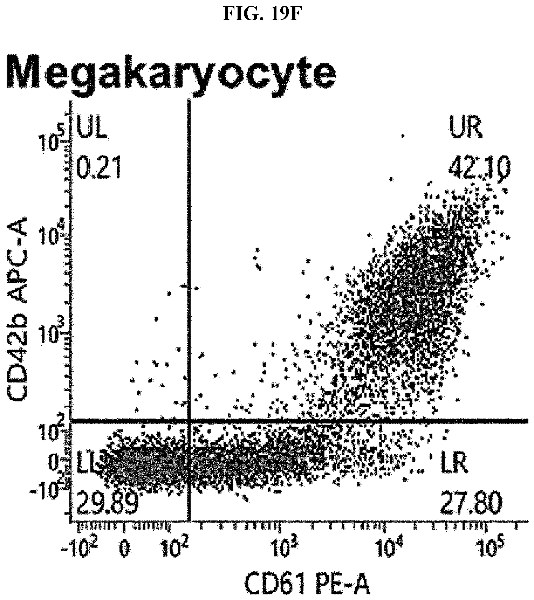

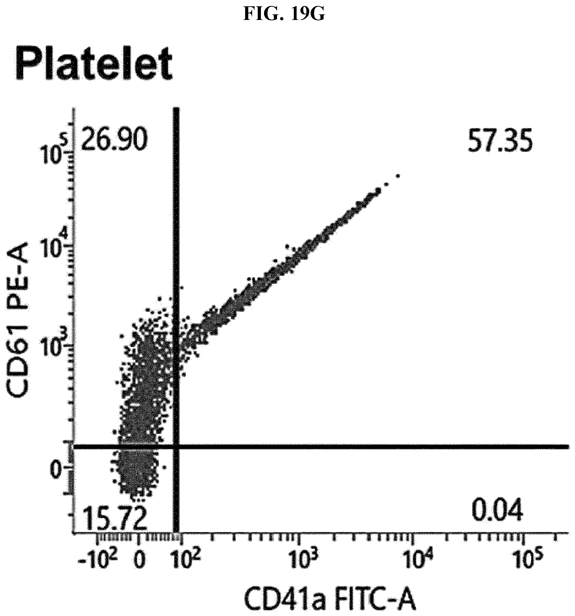

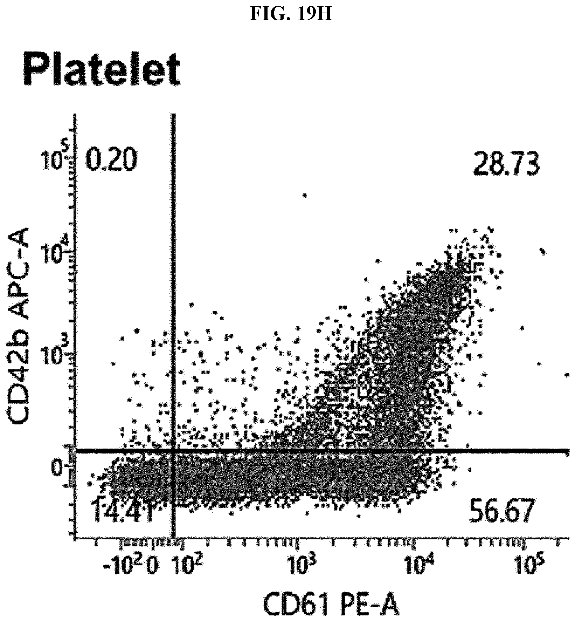

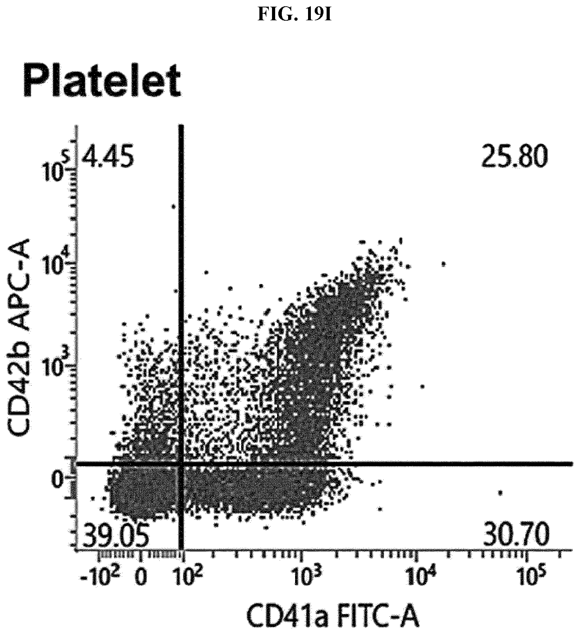

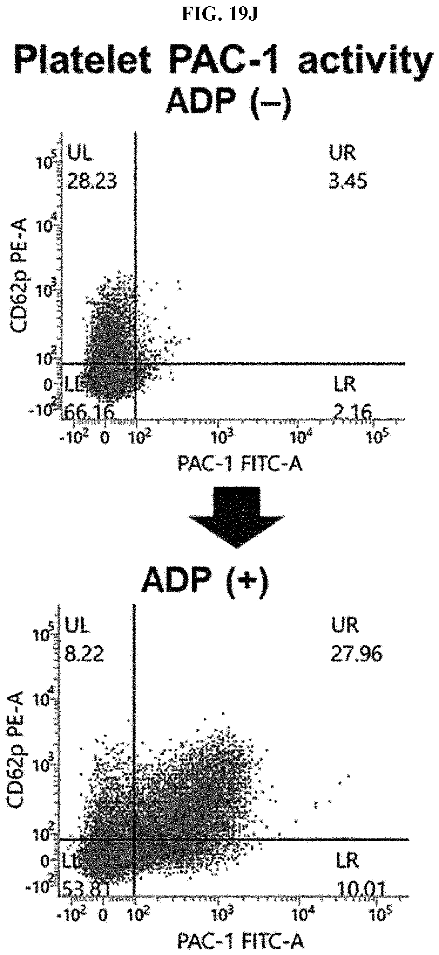

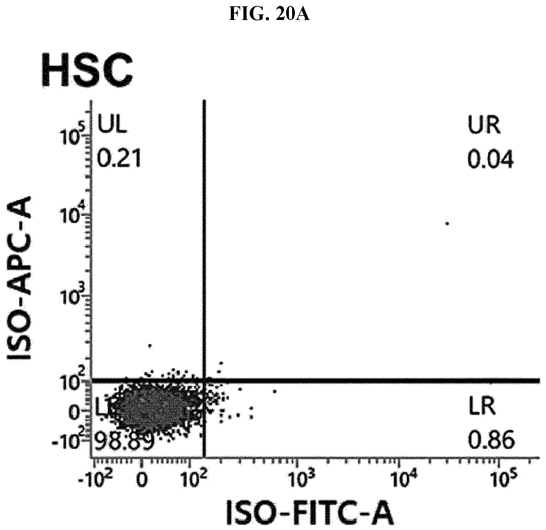

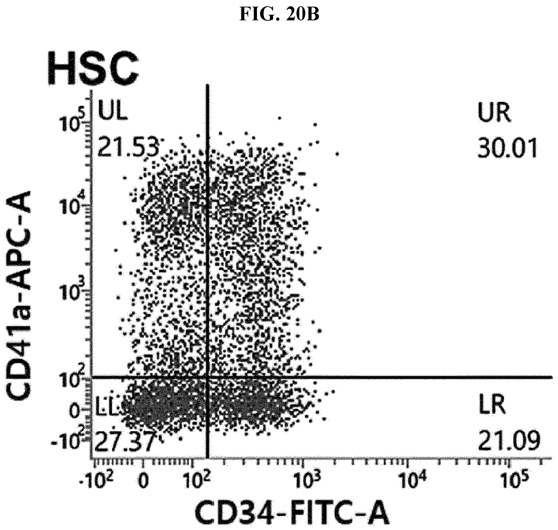

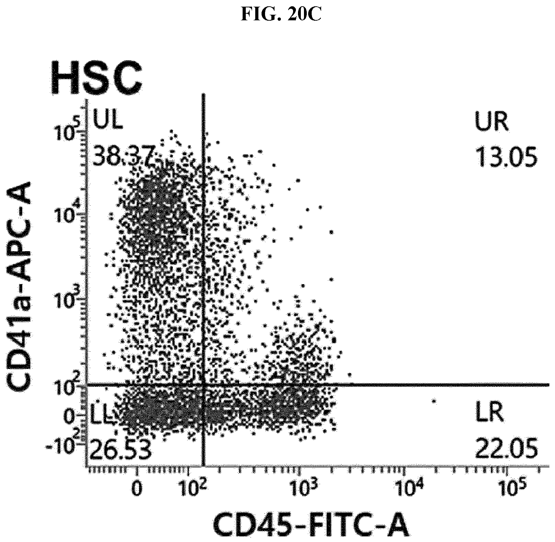

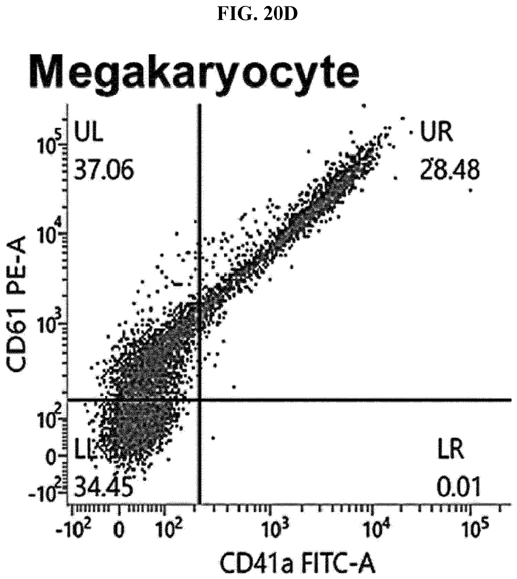

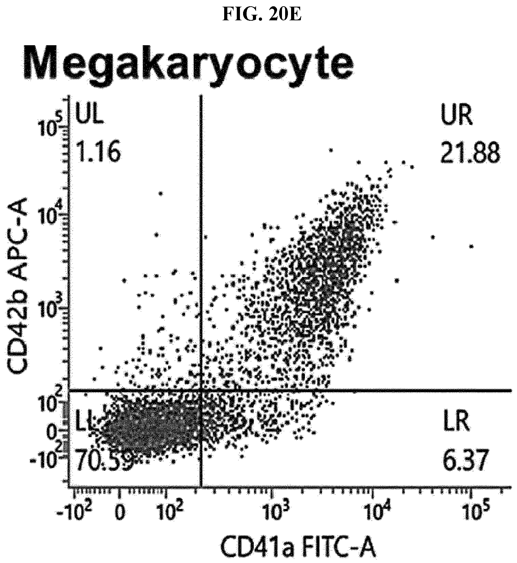

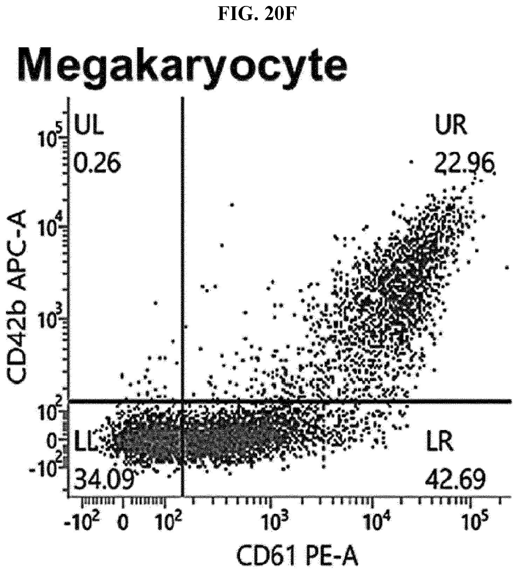

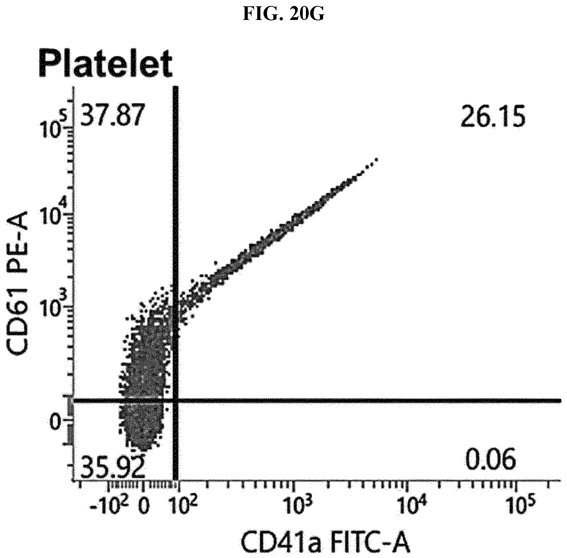

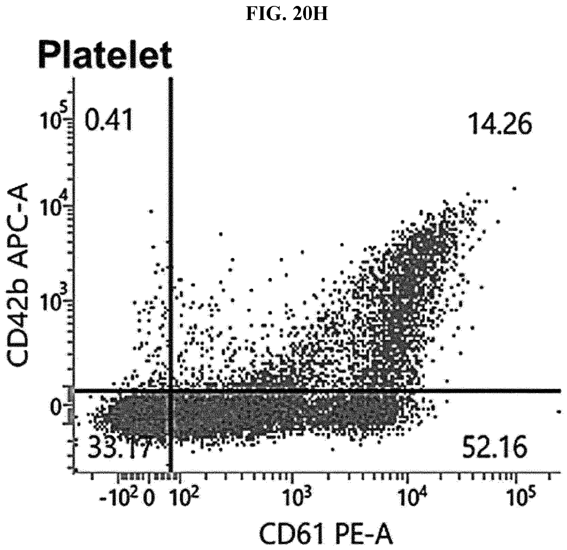

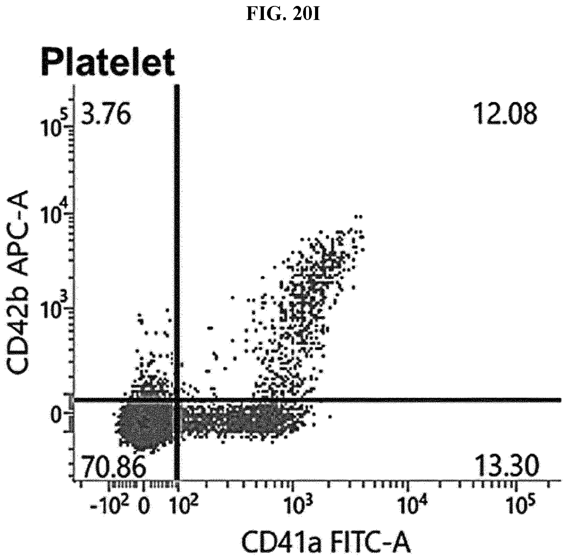

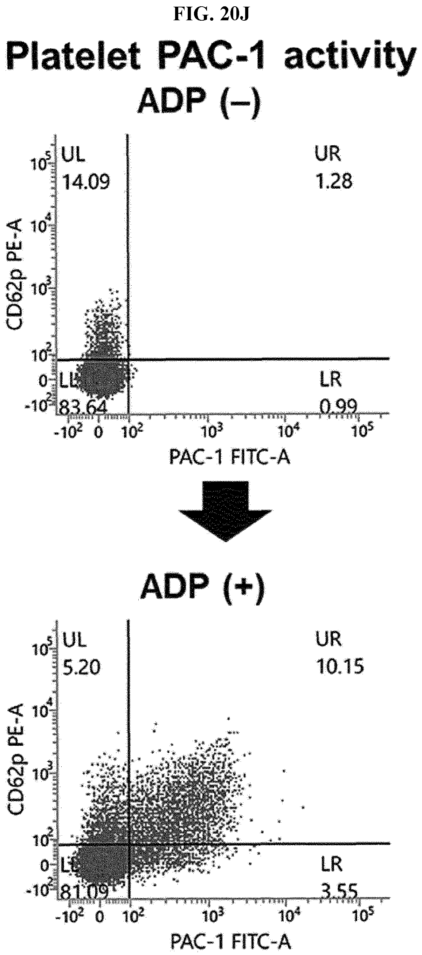

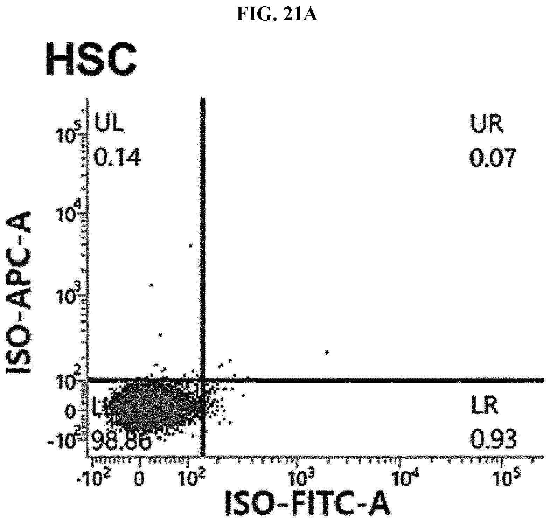

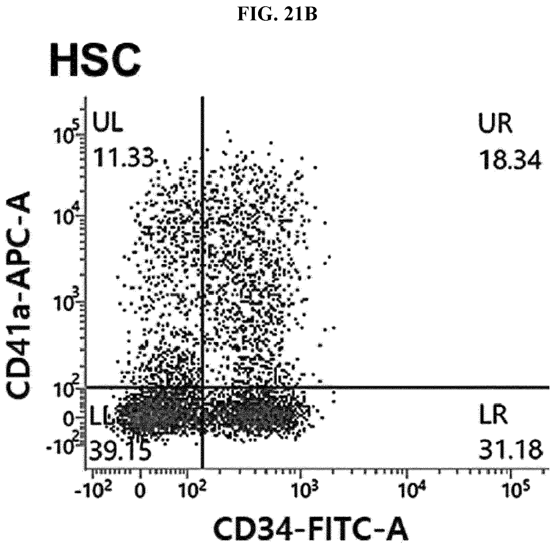

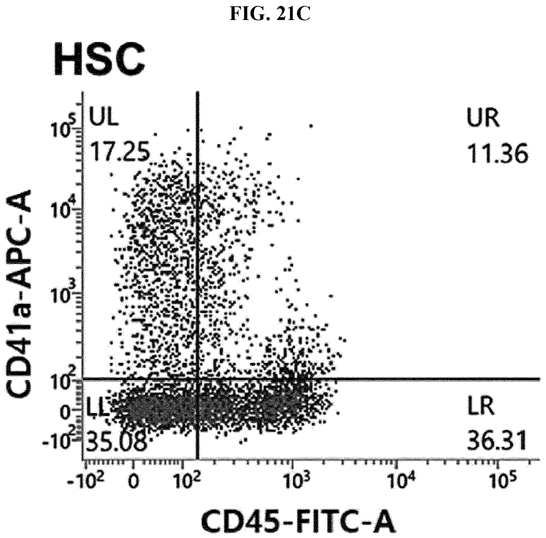

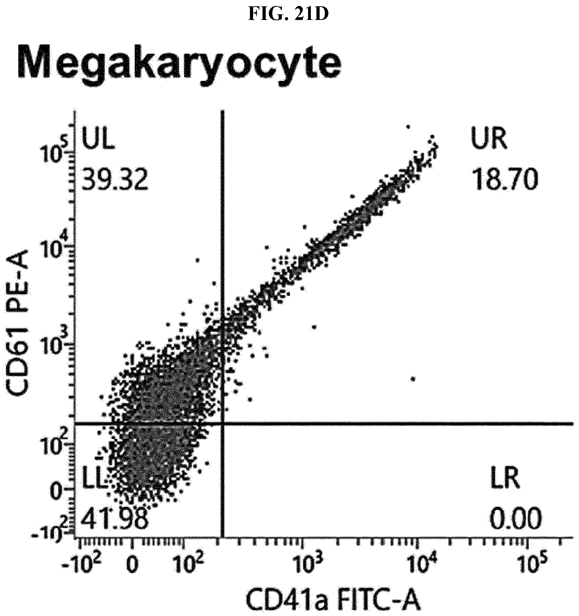

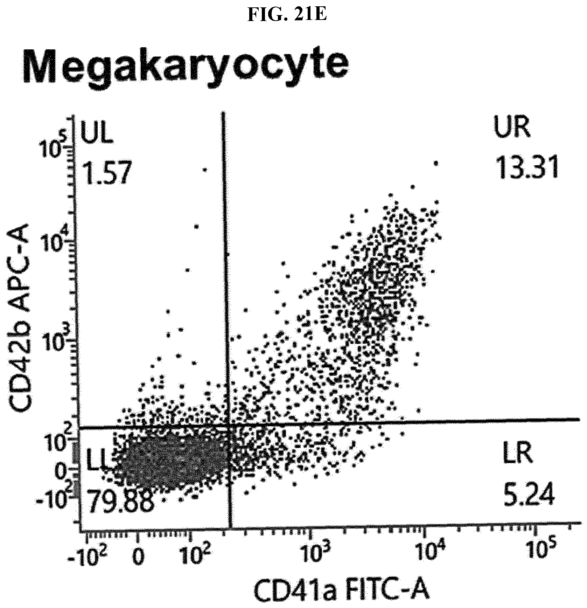

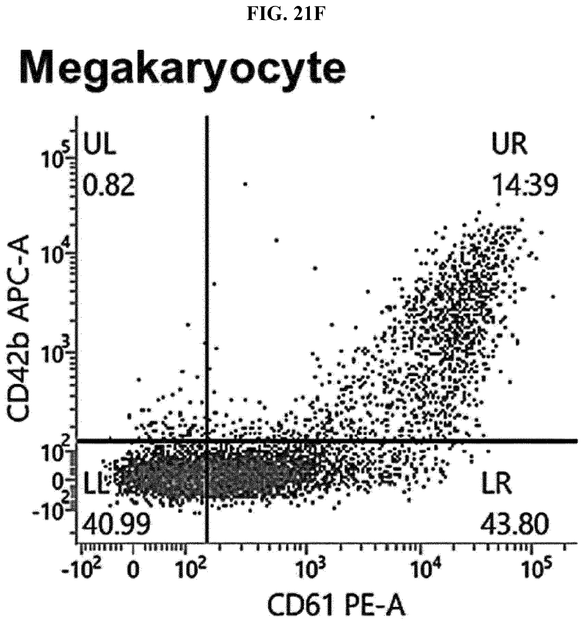

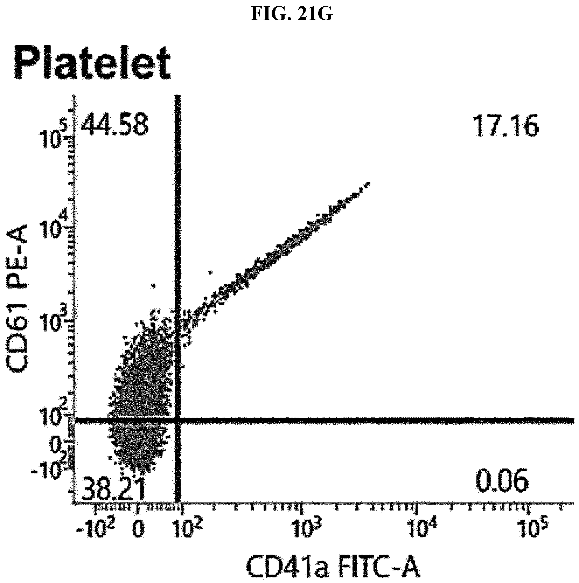

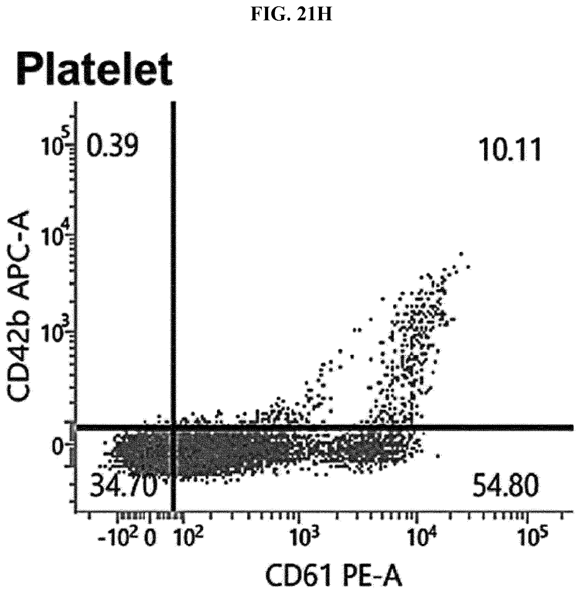

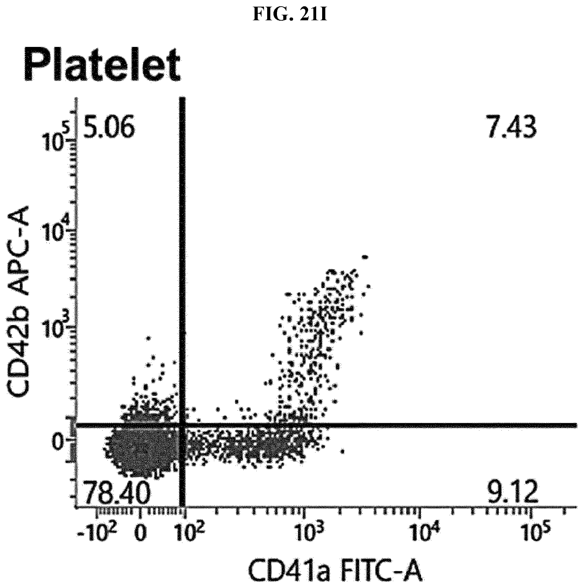

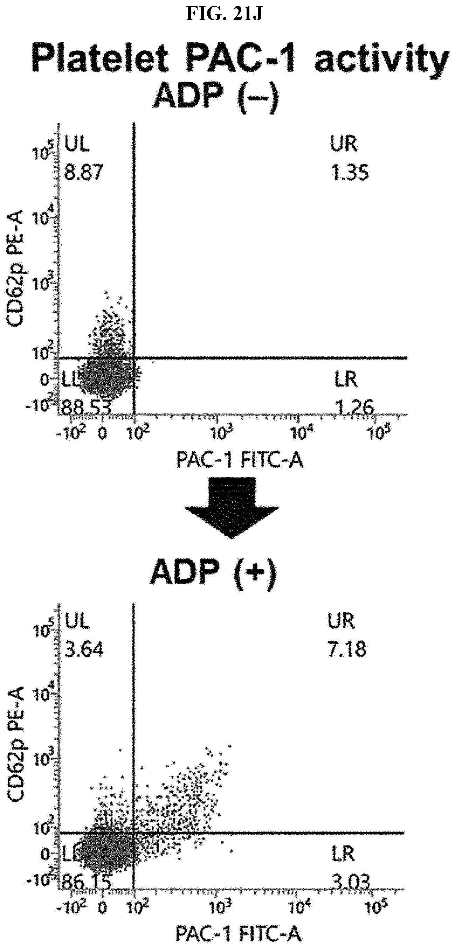

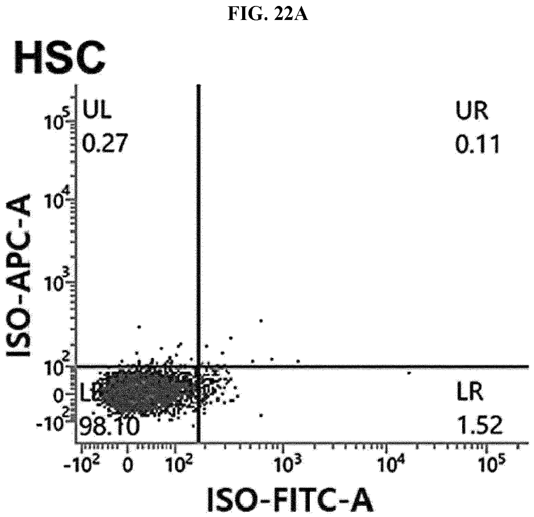

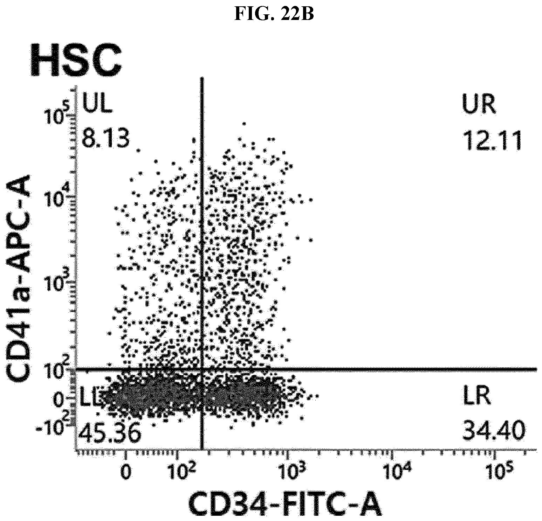

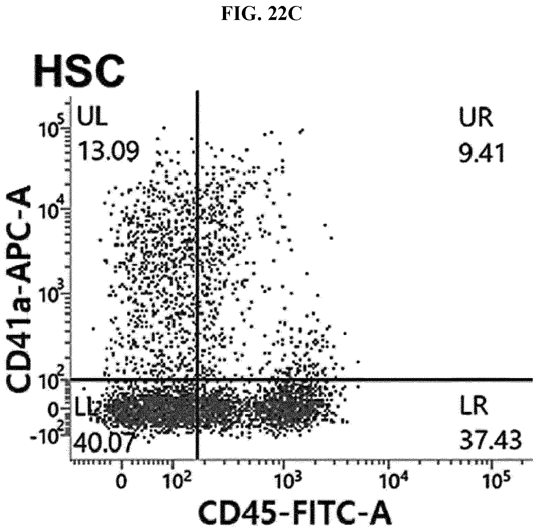

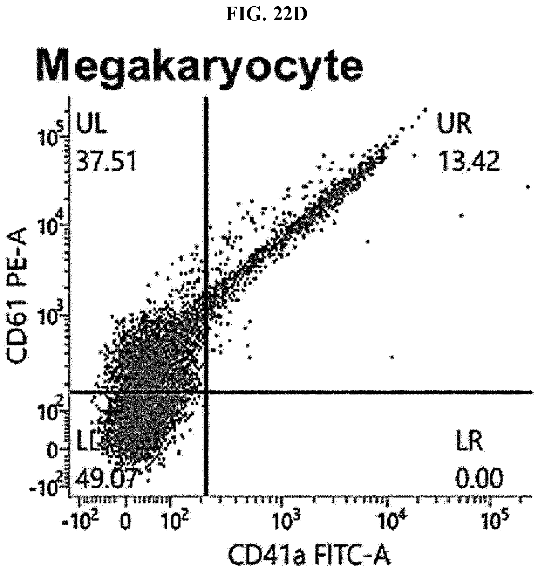

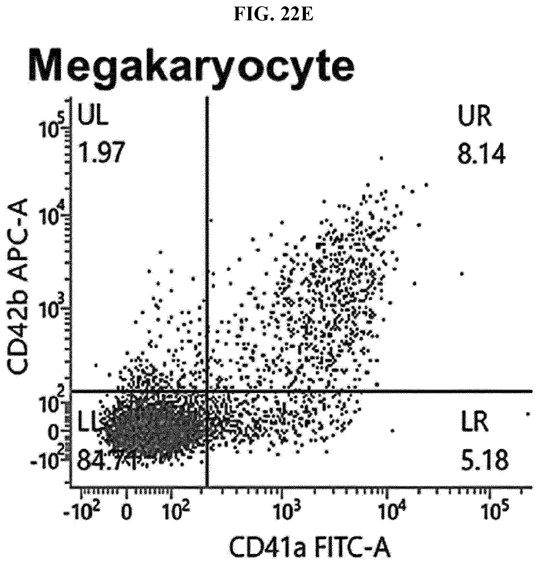

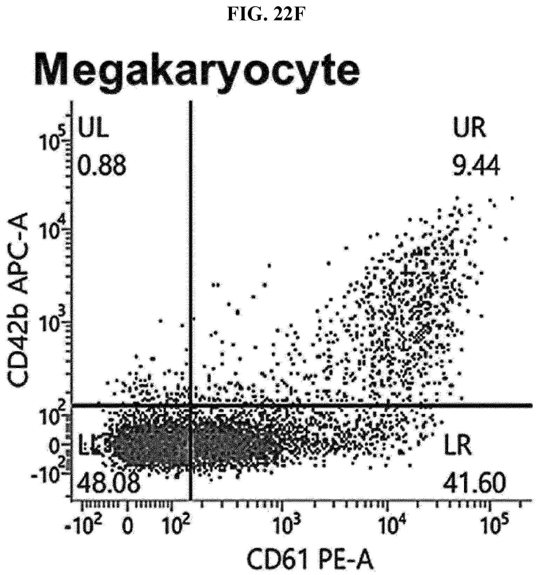

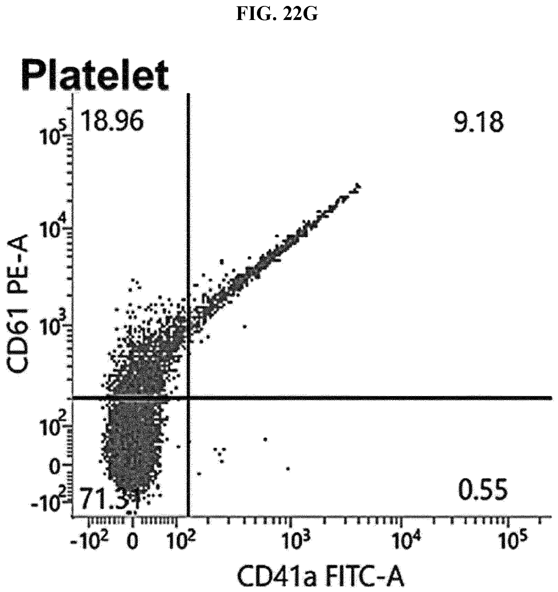

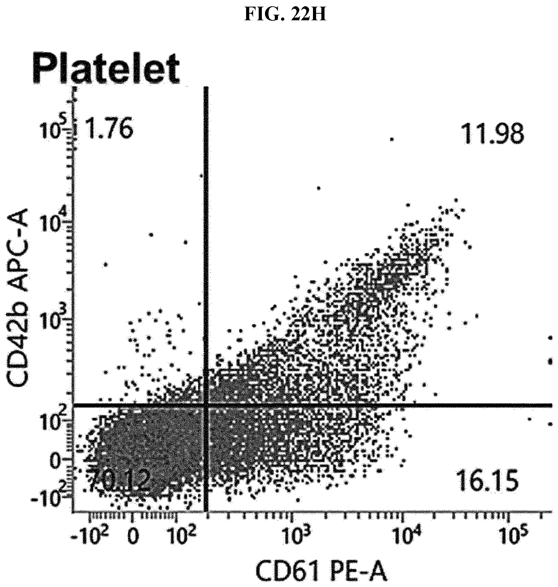

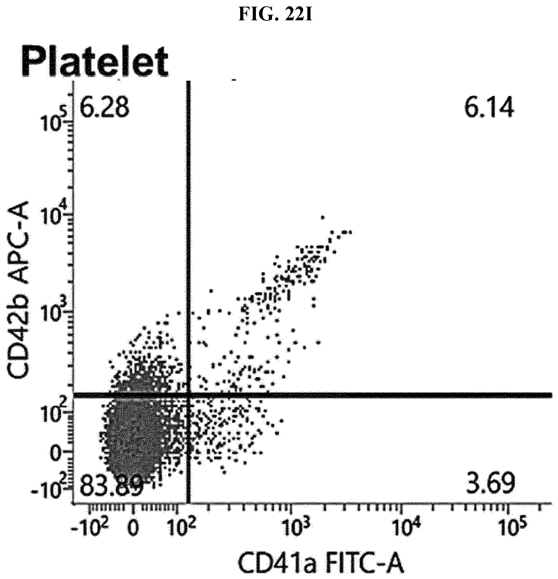

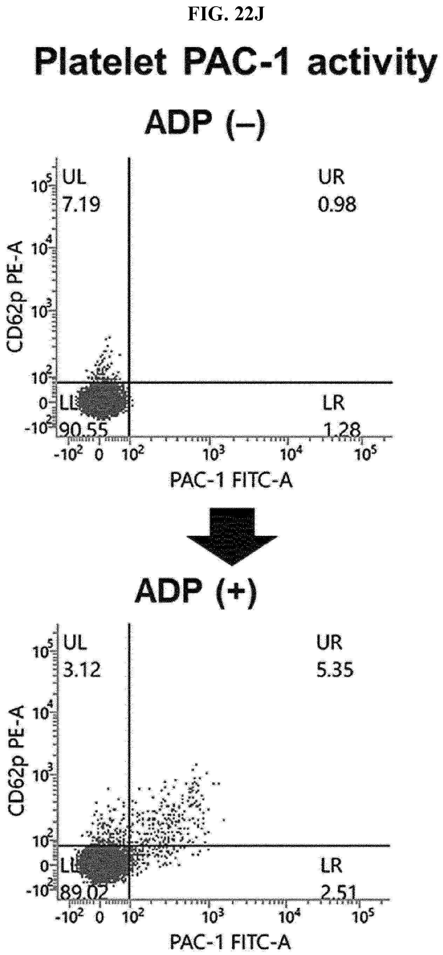

As a result, Examples 3 to 8 showed positive for the expression of megakaryocyte and platelet specific markers (FIG. 17 to 22). This confirms that the floating cell populations of Preparation Examples 3-1 to 3-6 are capable of producing megakaryocytes and platelets efficiently.

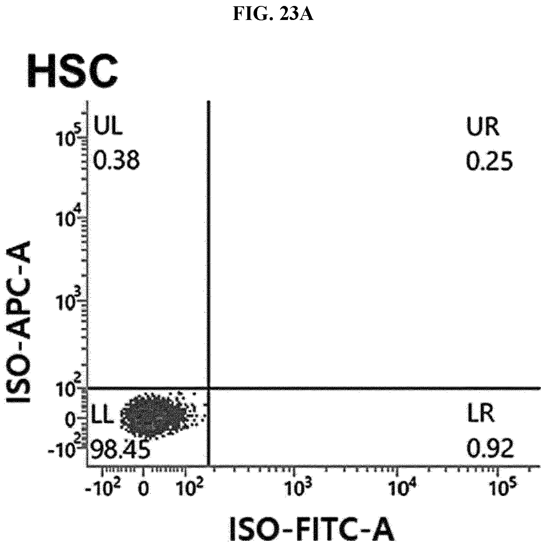

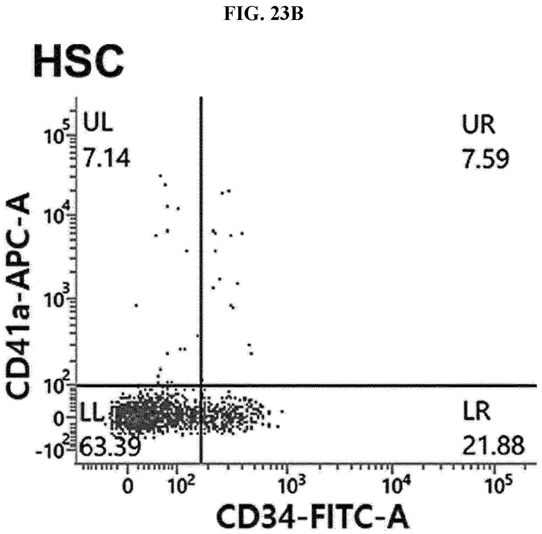

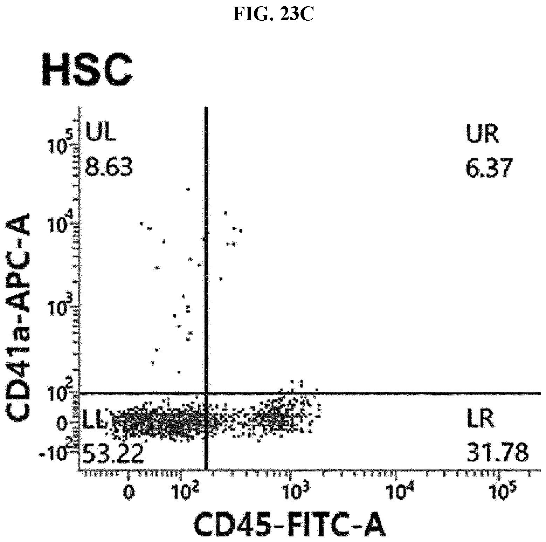

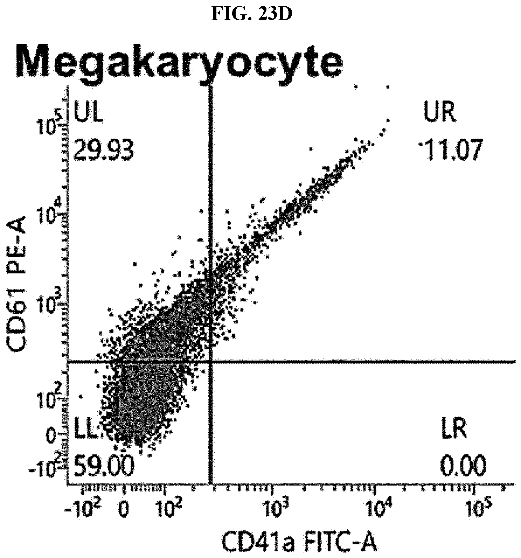

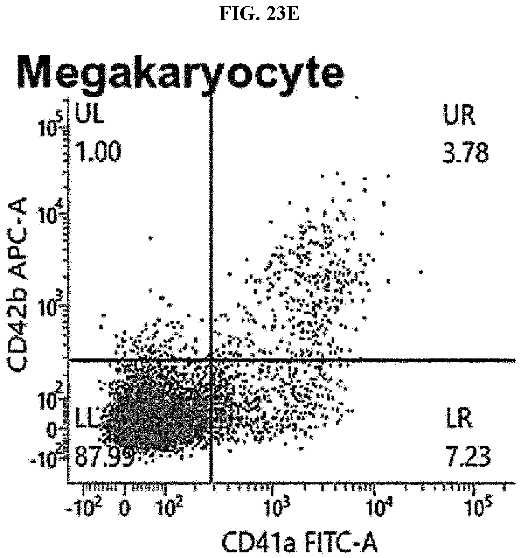

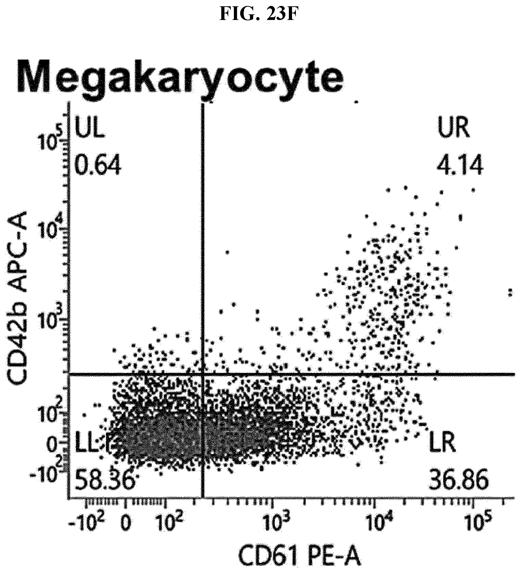

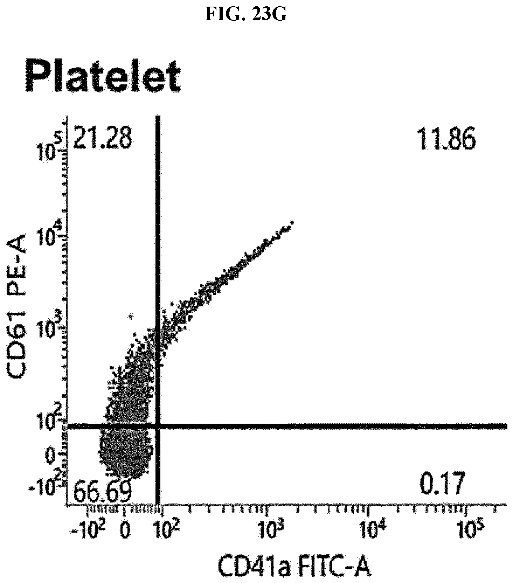

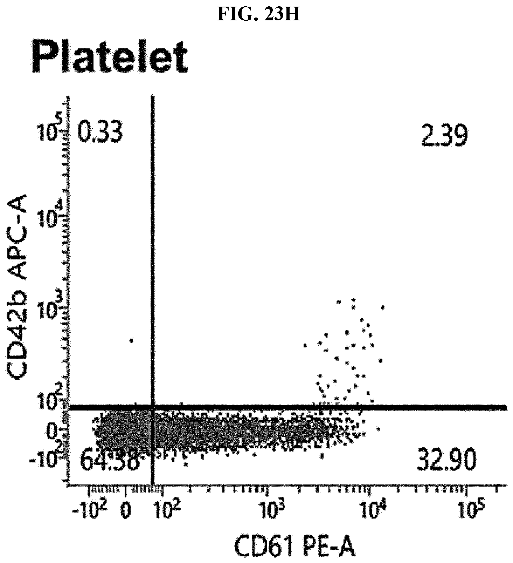

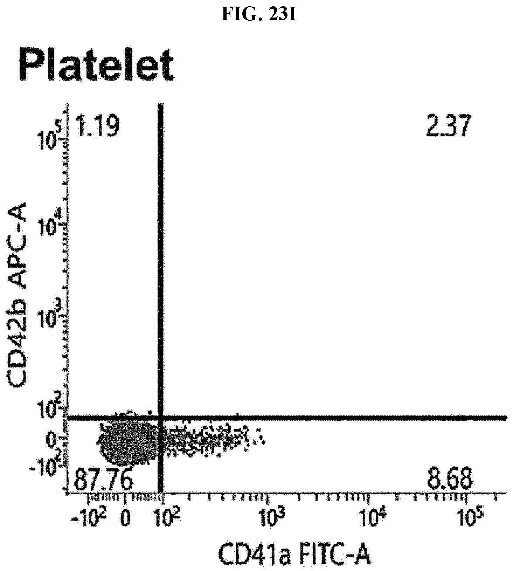

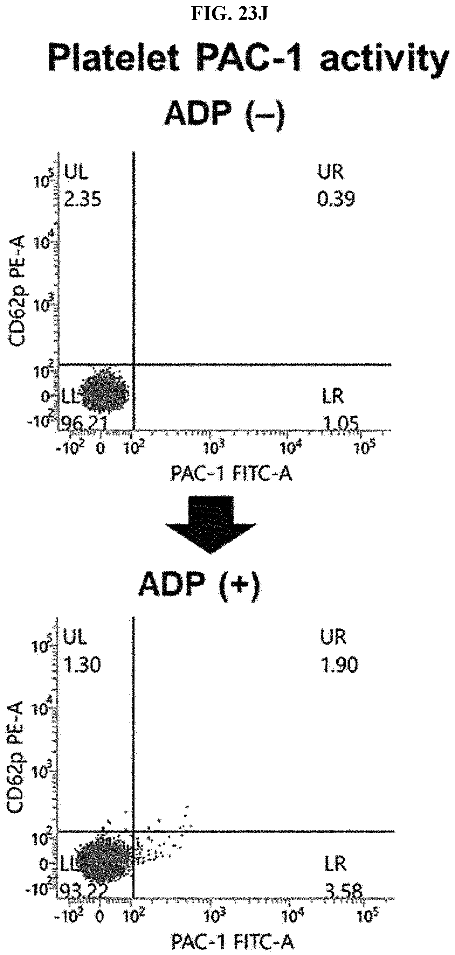

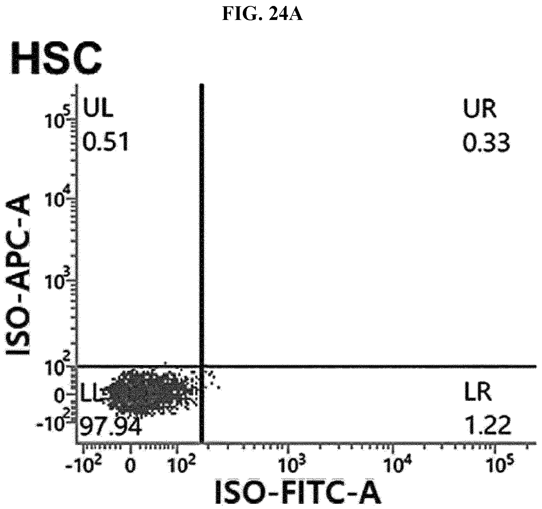

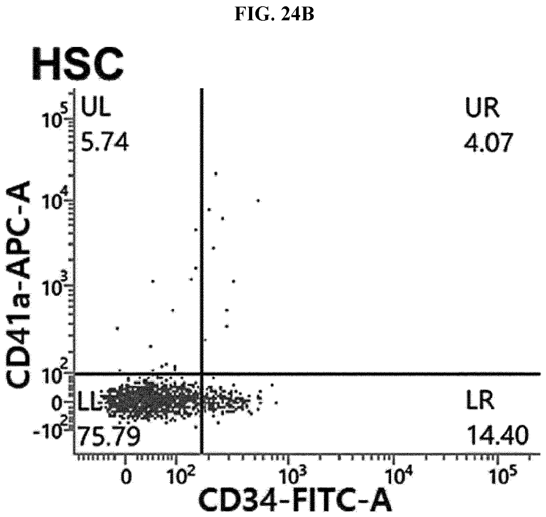

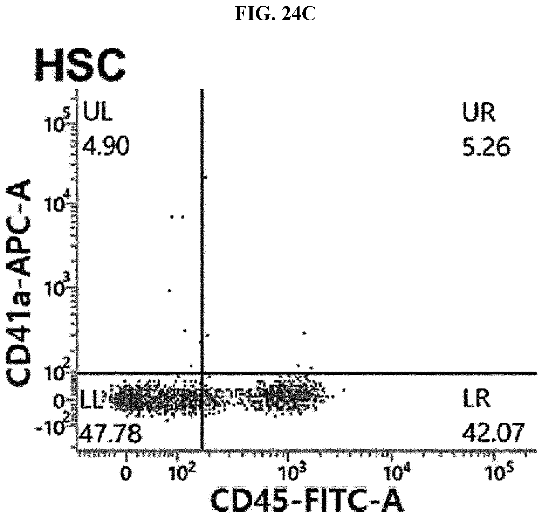

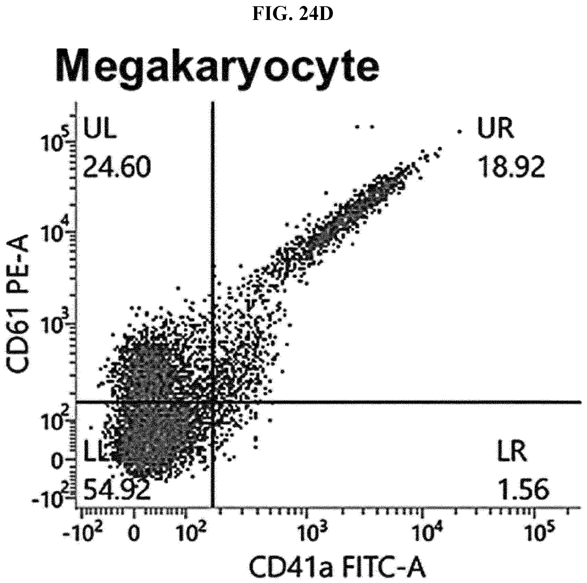

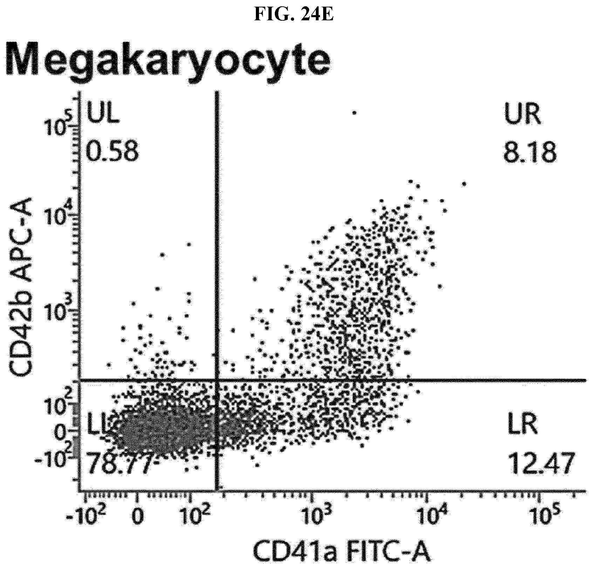

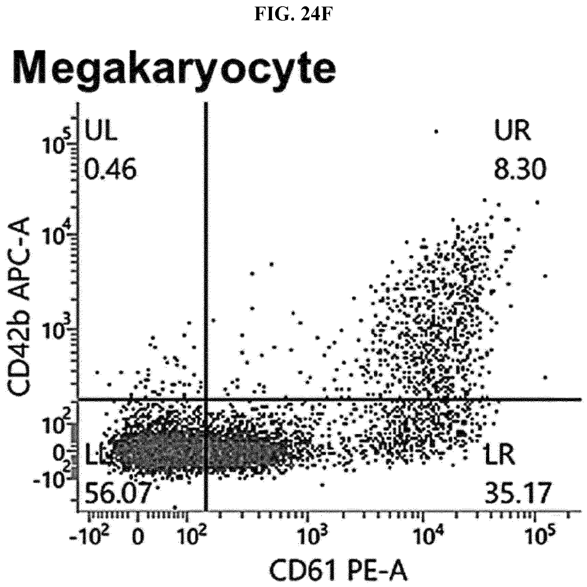

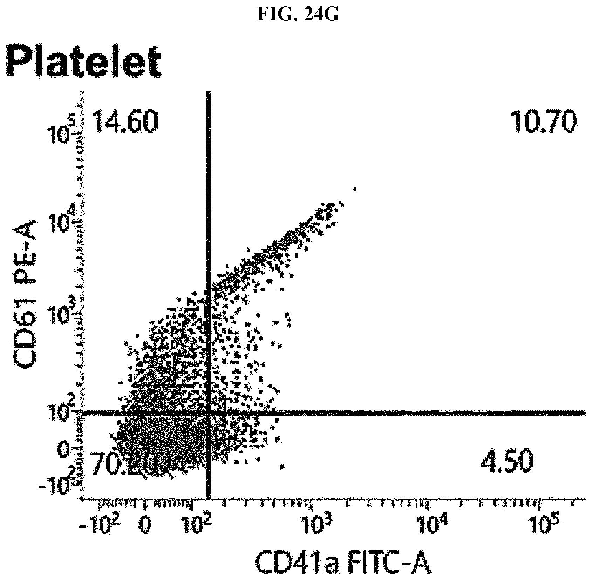

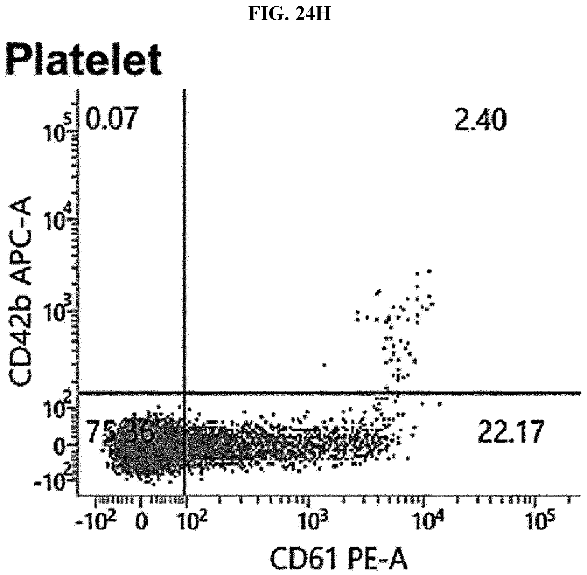

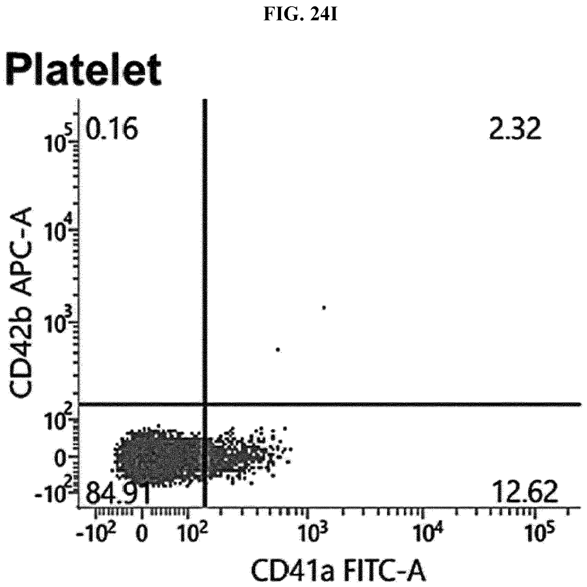

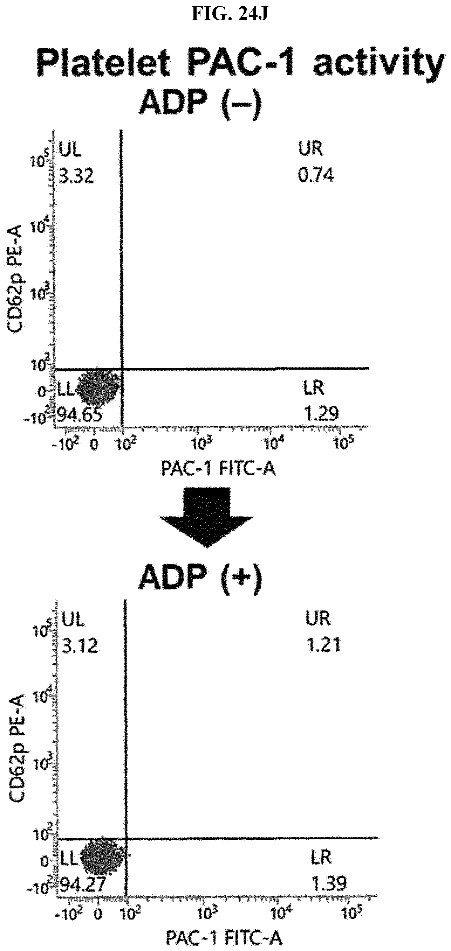

In contrast, Comparative Examples 3 and 4 showed almost no expression of the surface marker of megakaryocytes and platelets. Consequently, it was confirmed that the floating cell populations of Comparative Preparation Examples 3-1 and 3-2 showed almost no differentiation into megakaryocytes, and could not substantially produce platelets (FIG. 23 and 24).

### (2) Confirmation of Platelet Function by ADP Treatment

The following experiment was carried out to confirm whether Examples 3 to 8 and Comparative Examples 3 and 4, which had undergone the megakaryocyte maturation step, were platelets with normal function.

The cell culture from Preparation Examples 3-1 to 3-6 and Comparative Preparation Examples 3-1 and 3-2, which had undergone the megakaryocyte maturation step, were centrifuged. The precipitated megakaryocytes were removed. The suspended platelets were centrifuged again at 2000 xg for 15 minutes. To collect the platelets that had precipitated by centrifugation, the supernatant was removed and a small amount of PBS was used to resuspend the cells.

For PAC-1 analysis, 500 µM ADP was added to the platelets to react at room temperature for 15 minutes, and then reacted with anti-PAC-1-FITC and anti-CD62p-PE antibodies from BD for 30 minutes. FACS analysis was carried out immediately on the reacted platelets.

PAC-1 is what appears when CD41a and CD61 form a complex as platelets are activated. CD62p (p-selectin) is a surface molecule that is expressed increasingly as platelets are activated and acts as a place where leukocytes can bind to platelets.

Examples 3 to 8 were activated by the treatment with ADP and the expression of PAC-1 and CD62p was increased. This confirmed that Examples 3 to 8 were platelets having normal function (FIG. 17 to 22). In contrast, Comparative Examples 3 and 4 exhibited almost no platelet activation (FIG. 23 and 24).

As described above, it was confirmed that when the subsequent steps are performed with a floating cell population obtained from a culture when the number of CD41a⁻ cells is at least 15% of the total number of cells, the efficiency of differentiation into megakaryocytes and the efficiency of platelet production is increased.

## Claims

1. A method for producing platelets, comprising:
(S1) culturing pluripotent stem cells to obtain a culture containing hematopoietic stem cells;
(S2) obtaining a floating cell population from the culture when the number of CD41a⁻cells among the cells contained in the culture is at least 15% of the total number of cells; and
(S3) differentiating the floating cell population into megakaryocytes in a first medium.

2. The method for producing platelets according to claim 1, further comprising (S4) culturing and maturing the megakaryocytes in a second medium containing thrombopoietin.

3. The method for producing platelets according to claim 1, wherein (S1) comprises the following steps:
(S1a) culturing the pluripotent stem cells in a third medium containing a GSK3 inhibitor;
(S1b) culturing the cells cultured in the third medium in a fourth medium containing vascular endothelial growth factor and basic fibroblast growth factor; and
(S1c) culturing the cells cultured in the fourth medium in a fifth medium containing vascular endothelial growth factor; basic fibroblast growth factor; and a transforming growth factor beta signaling inhibitor.

4. The method for producing platelets according to claim 1, wherein the floating cell population has a CD45⁺ cell count of 50% or less of the total cell count.

5. The method for producing platelets according to claim 1, wherein the floating cell population has a CD34⁺ cell count of 45% or more of the total cell count.

6. The method for producing platelets according to claim 1, wherein the floating cell population has a CD41a⁻ cell count of 85% or less of the total cell count.

7. The method for producing platelets according to claim 1, further comprising (S0) seeding the pluripotent stem cells on the bottom of a culture vessel at 2,000 to 20,000 cells/cm².

8. The method for producing platelets according to claim 1, wherein the culture further comprises hematopoietic progenitor cells and megakaryocyte progenitor cells.

9. The method for producing platelets according to claim 1, wherein the first medium comprises thrombopoietin, stem cell factor, interleukin-3, and interleukin-6.

10. The method for producing platelets according to claim 1, wherein, in (S2), the floating cell population is obtained from the culture when the number of CD41a⁺ cells among the cells contained in the culture is 40 to 85% of the total number of cells.

11. The method for producing platelets according to claim 1, wherein, in (S2), the floating cell population is obtained from the culture when the number of CD34⁺ cells among the cells contained in the culture is 45 to 80% of the total number of cells.

12. The method for producing platelets according to claim 1, wherein, in (S2), the floating cell population is obtained from the culture when the number of CD45⁺ cells among the cells contained in the culture is 1 to 50% of the total number of cells.

13. The method for producing platelets according to claim 1, wherein the pluripotent stem cells are human induced pluripotent stem cells.

14. The method for producing platelets according to claim 4, wherein the floating cell population has a proportion of CD45⁺ cells lower than a proportion of CD41a+ cells.

15. A method for producing a blood product, comprising a step of mixing platelets produced by the method of any one of claims 1 to 14 with other blood components.
